# EUROPÄISCHE PATENTSCHRIFT

(11) **EP 2 486 146 B1**
(45) Veröffentlichungstag und Bekanntmachung des Hinweises auf die Patenterteilung: **06.11.2013**
(21) Anmeldenummer: 10757568.0
(22) Anmeldetag: 09.09.2010
(51) Int. Cl.: C12Q 1/68

(54) **VERFAHREN ZUR DETEKTION VON GENVERÄNDERUNGEN MITTELS ASYMMETRISCHER PCR UND BLOCKIERUNGSMITTELN**
METHOD FOR DETECTING GENE MODIFICATIONS BY MEANS OF ASYMMETRICAL PCR AND BLOCKING AGENTS
PROCÉDÉ DE DÉTECTION DE MODIFICATIONS GÉNÉTIQUES AU MOYEN DE LA PCR ASYMÉTRIQUE ET D'AGENTS BLOQUANTS

(30) Priorität: 09.10.2009 DE 102009049001; 15.03.2010 DE 102010011533; 29.06.2010 DE 102010025496
(43) Veröffentlichungstag der Anmeldung: 15.08.2012
(73) Patentinhaber: Universität Duisburg-Essen, 45141 Essen (DE)
(72) Erfinder: SCHULER, Martin, 45133 Essen (DE); BREITENBÜCHER, Frank, 45468 Mülheim (DE); HOFFARTH, Sandra, 45468 Mülheim (DE); STERGAR, Sarah-Luise, 45894 Gelsenkirchen (DE)
(74) Vertreter: Von Rohr
(86) Internationale Anmeldenummer: PCT/EP2010/005532
(87) Internationale Veröffentlichungsnummer: WO 2011/042104

(56) Entgegenhaltungen:
- WO-A1-2008/009740
- HOFFARTH, S., STERGAR, S.-L., BREITENBUECHER, F.: "Hochsensitiver Nachweis von Mutationen des humanen EGF-Rezeptors bei Patienten mit nicht-kleinzelligem Lungenkarzinom", MTA DIALOG, [Online] Bd. 11, Nr. 1, Januar 2010 (2010-01), Seiten 26-29, XP009141148, Darmstadt Gefunden im Internet: URL:http://www.mta-dialog.de/suche/Fachbei traegeTumordiagnostik.htm> [gefunden am 2010-11-15]
- NAGAI YOSHIAKI ET AL: "Genetic heterogeneity of the epidermal growth factor receptor in non-small cell lung cancer cell lines revealed by a rapid and sensitive detection system, the peptide nucleic acid-locked nucleic acid PCR clamp", CANCER RESEARCH, AMERICAN ASSOCIATION FOR CANCER REREARCH, US, Bd. 65, Nr. 16, 1. August 2005 (2005-08-01), Seiten 7276-7282, XP002413948, ISSN: 0008-5472, DOI: DOI:10.1158/0008-5472.CAN-05-0331
- KREUZER K-A ET AL: "Preexistence and evolution of imatinib mesylate-resistant clones in chronic myelogenous leukemia detected by a PNA-based PCR clamping technique", ANNALS OF HEMATOLOGY, BERLIN, DE, Bd. 82, Nr. 5, 1. Mai 2003 (2003-05-01), Seiten 284-289, XP002464825, ISSN: 0939-5555, DOI: DOI:10.1007/S00277-003-0690-5
- SENESCAU ALICE ET AL: "Use of a locked-nucleic-acid oligomer in the clamped-probe assay for detection of a minority Pfcrt K76T mutant population of Plasmodium falciparum.", JOURNAL OF CLINICAL MICROBIOLOGY JUL 2005 LNKD- PUBMED:16000452, Bd. 43, Nr. 7, Juli 2005 (2005-07), Seiten 3304-3308, XP002609843, ISSN: 0095-1137
- KAWAI Y ET AL: "Sensitive detection of EGFR mutations using a competitive probe to suppress background in the SMart Amplification Process", BIOLOGICALS, ACADEMIC PRESS LTD., LONDON, GB, Bd. 36, Nr. 4, 1. Juli 2008 (2008-07-01), Seiten 234-238, XP022795226, ISSN: 1045-1056, DOI: DOI:10.1016/J.BIOLOGICALS.2008.01.003 [gefunden am 2008-04-28]
- WILLMORE-PAYNE CARLYNN ET AL: "The use of EGFR exon 19 and 21 unlabeled DNA probes to screen for activating mutations in non-small cell lung cancer.", JOURNAL OF BIOMOLECULAR TECHNIQUES : JBT JUL 2008 LNKD- PUBMED:19137110, Bd. 19, Nr. 3, Juli 2008 (2008-07), Seiten 217-224, XP002609844, ISSN: 1943-4731
- ERALI M ET AL: "High resolution melting applications for clinical laboratory medicine", EXPERIMENTAL AND MOLECULAR PATHOLOGY, ACADEMIC PRESS, US, Bd. 85, Nr. 1, 1. August 2008 (2008-08-01) , Seiten 50-58, XP022850522, ISSN: 0014-4800, DOI: DOI:10.1016/J.YEXMP.2008.03.012 [gefunden am 2008-04-13]
- ASANO HIROAKI ET AL: "Detection of EGFR gene mutation in lung cancer by mutant-enriched polymerase chain reaction assay.", CLINICAL CANCER RESEARCH : AN OFFICIAL JOURNAL OF THE AMERICAN ASSOCIATION FOR CANCER RESEARCH 1 JAN 2006 LNKD- PUBMED:16397022, Bd. 12, Nr. 1, 1. Januar 2006 (2006-01-01) , Seiten 43-48, XP002609845, ISSN: 1078-0432
- KIMURA HIDEHARU ET AL: "Detection of epidermal growth factor receptor mutations in serum as a predictor of the response to gefitinib in patients with non-small-cell lung cancer", CLINICAL CANCER RESEARCH, THE AMERICAN ASSOCIATION FOR CANCER RESEARCH, US, Bd. 12, Nr. 13, 1. Juli 2006 (2006-07-01), Seiten 3915-3921, XP002413945, ISSN: 1078-0432, DOI: DOI:10.1158/1078-0432.CCR-05-2324
- PAO WILLIAM ET AL: "Epidermal growth factor receptor mutation testing in lung cancer: searching for the ideal method.", CLINICAL CANCER RESEARCH : AN OFFICIAL JOURNAL OF THE AMERICAN ASSOCIATION FOR CANCER RESEARCH 1 SEP 2007 LNKD- PUBMED:17785543, Bd. 13, Nr. 17, 1. September 2007 (2007-09-01), Seiten 4954-4955, XP002609846, ISSN: 1078-0432
- YATABE YASUSHI ET AL: "Epidermal growth factor receptor mutations in lung cancers", PATHOLOGY INTERNATIONAL, CARLTON SOUTH, AU, Bd. 57, Nr. 5, 1. Mai 2007 (2007-05-01), Seiten 233-244, XP002569947, ISSN: 1320-5463, DOI: DOI:10.1111/J.1440-1827.2007.02098.X [gefunden am 2007-04-16]
- SHARMA S V ET AL: "Epidermal growth factor receptor mutations in lung cancer", NATURE REVIEWS. CANCER, NATUR PUBLISHING GROUP, LONDON, GB, Bd. 7, 1. März 2007 (2007-03-01), Seiten 169-181, XP002556732, ISSN: 1474-175X, DOI: DOI:10.1038/NRC2088

### SEQUENCE LISTING

<110> Universitaet Duisburg-Essen
<120> Verfahren zur Detektion von Genveraenderungen, insbesondere Mutationen
<130> 10.2884.6.do
<160> 11
<170> PatentIn version 3.3
<210> 1
   <211> 1210
   <212> PRT
   <213> Homo sapiens
<400> 1
<210> 2
   <211> 26
   <212> DNA
   <213> Artificial Sequence
<220>
   <223> PCR-Oligonukleotid, spezifisch fuer das korrespondierende Gen des humanen epidermalen Wachstumsfakor-Rezeptors (EGFR)
<400> 2
   gactccgact cctcctttat ccaatg 26
<210> 3
   <211> 21
   <212> DNA
   <213> Artificial Sequence
<220>
   <223> PCR-Oligonukleotid, spezifisch fuer das korrespondierende Gen des humanen epidermalen Wachstumsfakor-Rezeptors (EGFR)
<400> 3
   cacacaccag ttgagcaggt a 21
<210> 4
   <211> 19
   <212> DNA
   <213> Artificial Sequence
<220>
   <223> PCR-Oligonukleotid, spezifisch fuer das korrespondierende Gen des humanen epidermalen Wachstumsfakor-Rezeptors (EGFR)
<400> 4
   gctcagagcc tggcatgaa 19
<210> 5
   <211> 19
   <212> DNA
   <213> Artificial Sequence
<220>
   <223> PCR-Oligonukleotid, spezifisch fuer das korrespondierende Gen des humanen epidermalen Wachstumsfakor-Rezeptors (EGFR)
<400> 5
   catcctcccc tgcatgtgt 19
<210> 6
   <211> 20
   <212> DNA
   <213> Artificial Sequence
<220>
   <223> Real-Time PCR-Sonde, spezifisch fuer die Punkmutation T790M des EGFR-Gens (Sensorsonde)
<220>
   <221> misc_binding
   <222> (1)..(1)
   <223> FRET-Komponente, bevorzugterweise Fluoreszenzfarbstoff
<400> 6
   ggcatgagct gcatgatgag 20
<210> 7
   <211> 23
   <212> DNA
   <213> Artificial Sequence
<220>
   <223> Real-Time PCR-Sonde, spezifisch fuer die Punkmutation T790M des EGFR-Gens (Ankersonde)
<220>
   <221> misc_binding
   <222> (23)..(23)
   <223> FRET-Komponente, bevorzugterweise Fluoreszenzfarbstoff
<400> 7
   cacggtggag gtgaggcaga tgc 23
<210> 8
   <211> 15
   <212> DNA
   <213> Artificial Sequence
<220>
   <223> Real-Time PCR-Sonde, spezifisch fuer die Punkmutation L858R des EGFR-Gens (Ankersonde)
<220>
   <221> misc_binding
   <222> (1)..(1)
   <223> FRET-Komponente, bevorzugterweise Fluoreszenzfarbstoff
<400> 8
   gtttggcccg cccaa 15
<210> 9
   <211> 30
   <212> DNA
   <213> Artificial Sequence
<220>
   <223> Real-Time PCR-Sonde, spezifisch fuer die Punkmutation L858R des EGFR-Gens (Ankersonde)
<220>
   <221> misc_binding
   <222> (30)..(30)
   <223> FRET-Komponente, bevorzugterweise Fluoreszenzfarbstoff
<400> 9
   gcatggtatt ctttctcttc cgcacccagc 30
<210> 10
   <211> 14
   <212> DNA
   <213> Artificial Sequence
<220>
   <223> Blockierungsmittel nach dem Prinzip der locked nucleic acid, einsetzbar zur Real-Time PCR, spezifisch fuer einen Abschnitt auf dem wildtypischen EGFR-Gen
<220>
   <221> modified_base
   <222> (1)..(14)
   <223> Modifizierung zur Herstellung von Blockiermitteln nach Prinzip der locked nucleic acid, dazu werden das C2 und das C4-Atom der Ribose/Desoxyribose ueber eine Sauerstoff-Methylengruppe miteinder verbunden
<400> 10
   tgagctgcgt gatg 14
<210> 11
   <211> 15
   <212> DNA
   <213> Artificial Sequence
<220>
   <223> Blockierungsmittel nach dem Prinzip der locked nucleic acid, einsetzbar zur Real-Time PCR, spezifisch fuer einen Abschnitt auf dem wildtypischen EGFR-Gen
<220>
   <221> modified_BASE
   <222> (1)..(15)
   <223> Modifizierung zur Herstellung von Blockiermitteln nach Prinzip der locked nucleic acid, dazu werden das C2 und das C4-Atom der Ribose/Desoxyribose ueber eine Sauerstoff-Methylengruppe miteinder verbunden
<400> 11
   gccagcccaa aatct 15

## Beschreibung

Die vorliegende Erfindung betrifft den Bereich des Nachweises bzw. der Detektion von Genveränderungen, insbesondere Mutationen, in genomischer DNA, wobei die Genveränderung bzw. Mutation insbesondere im Zusammenhang mit einer Tumor- und/oder Krebserkrankung, wie einem Bronchialkarzinom, in Verbindung steht. Auf Basis des Nachweises bzw. der Detektion der Genveränderung können Therapieansätze bzw. -maßnahmen zur gezielten Behandlung der Krebs- bzw. Tumorerkrankung zweckgerichtet optimiert werden.

Insbesondere betrifft die vorliegende Erfindung ein Verfahren zum Nachweis bzw. zur Detektion mindestens einer Genveränderung, insbesondere Mutation, in einem Gen, vorzugsweise in einem Gen, welches für ein mit einer Tumor- und/oder Krebserkrankung in Verbindung stehenden Protein kodiert, nach dem Oberbegriff von Anspruch 1.

Zudem betrifft die vorliegende Erfindung eine Zusammensetzung, insbesondere zur Verwendung im Rahmen einer asymmetrischen Polymerase-Kettenreaktion, welche spezifische Komponenten aufweist, wobei die Zusammensetzung nach der Erfindung anwendungsfertig beispielsweise in Form einer wäßrigen Lösung bzw. Dispersion oder aber in Form räumlich voneinander getrennter Komponenten auf Basis eines Kit bzw. Kit-of-Parts vorliegen kann.

Schließlich betrifft die vorliegende Erfindung die Verwendung der erfindungsgemäßen Zusammensetzung zum Nachweis bzw. zur Detektion mindestens einer Genveränderung, insbesondere einer Mutation.

Krebs- bzw. Tumorerkrankungen sind nach den Erkrankungen des Herz/Kreislauf-Systems die zweithäufigste Todesursache in Deutschland. Sofern eine rechtzeitige Therapie begonnen wird oder die Tumorerkrankung erst im hohen Lebensalter auftritt und mitunter nur langsam voranschreitet, ist nicht jede Krebserkrankung tödlich. Die derzeitige Heilungsrate bei allen Krebserkrankungen beträgt durchschnittlich 30 bis 40 %, wobei sich diesbezüglich jedoch in Abhängigkeit von der konkreten Krebserkrankung deutliche Unterschiede ergeben. So zählen zum Beispiel Krebserkrankungen der Atemwege, insbesondere der Lunge, zu den nur schlecht therapierbaren Krebserkrankungen.

Bei Krebserkrankungen bzw. bei Tumorzellen ist die Abstimmung von Wachstum, Teilung und Zerstörung bzw. Apoptose im Zellverband gestört bzw. außer Kraft gesetzt. Oftmals werden körpereigene regulierende Signale nicht erkannt oder nicht bzw. fehlerhaft ausgeführt, was oftmals ursächlich auf genetische Defekte bzw. Genveränderungen, wie Mutationen, zurückzuführen ist. So können genetische Veränderungen, wie Mutationen, zu Veränderungen in der Struktur und in der Physiologie von durch die betroffenen Gene kodierten Proteinen führen, was ein Tumorwachstum induzieren bzw. fördern kann. So kann die Krebsentstehung bzw. Karzinogenese, insbesondere das primäre Krankheitsereignis, auf einer Veränderung des Erbgutes beruhen, welche durch körpereigene Überwachungs- und Korrektursysteme nicht kompensiert werden und folglich beispielsweise im Rahmen von Zellteilungsprozessen auf nachfolgende Zellen übertragen werden kann, was mitunter zu der Entstehung eines Primärtumors führt.

Lungenkarzinome, welche synonym auch als Bronchialkarzinome, bronchogenes Karzinom bzw. Lungenkrebs bezeichnet werden, stellen eine bösartige Tumorerkrankung auf Basis entarteter Zellen insbesondere der Bronchien oder der Bronchiolen dar. Das Bronchial- bzw. Lungenkarzinom ist eine der häufigsten bösartigen Krebserkrankungen des Menschen und stellt eine der häufigsten durch Krebs bedingten Todesursachen in der westlichen Welt dar. So liegt die Zahl der Neuerkrankungen an Lungenkrebs in Deutschland bei etwa 50.000 Personen pro Jahr. Hauptursache für Lungenkrebserkrankungen ist das inhalative Rauchen. Daneben gibt es einige toxische Substanzen, wie Asbest oder Chrom, welche gleichermaßen Lungenkarzinome induzieren können. Aufgrund der in frühen Erkrankungsphasen mitunter ganz fehlenden oder nur unspezifischen Symptome werden die meisten Erstdiagnosen eines Lungenkarzinoms erst in späteren Stadien der Erkrankung gestellt, so daß eine der erfolgversprechendsten Therapieoption - eine vollständige operative Entfernung des Tumors - oftmals nicht mehr realisierbar ist, insbesondere auch aufgrund einer bereits voranschreitenden Metastasierung. Die Heilungsrate des Bronchialkarzinoms ist im allgemeinen sehr schlecht und liegt bei einer Fünfjahresüberlebensrate von unter 10 %; die Überlebenswahrscheinlichkeit nach zwei Jahren liegt unter 20 %.

Etwa ein Viertel aller bösartigen Tumore bzw. Malignome sind Bronchialkarzinome. Beim Mann ist das Bronchialkarzinom weltweit die häufigste Tumorerkrankung, in Deutschland ist sie die dritthäufigste nach dem Prostatakarzinom und dem kolorektalen Karzinom, jedoch liegt das Bronchialkarzinom als Ursache von Krebssterbefällen auf dem ersten Platz.

Aufgrund ihrer Histologie und des Krankheitsverlaufes werden Lungenkarzinome im allgemeinen in zwei Gruppen unterteilt, nämlich in ein kleinzelliges Lungenkarzinom (*Small Cell Lung Cancer* bzw. SCLC) einerseits und ein nichtkleinzelliges Lungenkarzinom (*Non Small Cell Lung Cancer* bzw. NSCLC) andererseits. Das nichtkleinzellige Lungenkarzinom bzw. NSCLC stellt mit einer Häufigkeit von 85 % der Lungekrebserkrankungen die größte Gruppe der Bronchialkarzinome dar. In Abhängigkeit von dem histologischen Befund kann das nichtkleinzellige Karzinom bzw. NSCLC in ein mitunter spindelzellig ausgebildetes Plattenepithelkarzinom, ein Adenokarzinom sowie in ein großzelliges Karzinom bzw. Riesenzellkarzinom differenziert werden.

Was die im Stand der Technik bekannten therapeutischen Ansätze zur Therapie des Lungenkarzinoms, insbesondere des kleinzelligen Lungenkarzinoms, anbelangt, so fokussieren diese maßgeblich auf einen therapeutischen Ansatz auf Basis einer Chemo- bzw. Radiotherapie. Derartige Therapien sind jedoch mit starken Nebenwirkungen verbunden und führen oftmals nicht zum gewünschten therapeutischen Erfolg. Auch platinbasierte Kombinationstherapien erreichen in Studien nur eine mediale Überlebensverlängerung von lediglich 10 bis 12 Monaten. Seit kurzer Zeit werden für Patienten mit einem diagnostizierten kleinzelligen Lungenkarzinom bzw. NSCLC Therapiealternativen zu der üblichen Behandlung mit Chemotherapeutika bereitgestellt. So werden Medikamente eingesetzt, die - im Gegensatz zu Zytostatika - spezifisch auf Tumorzellen wirken und, damit verbunden, auch deutlich weniger Nebenwirkungen aufweisen. Hierzu zählen insbesondere die unter den internationalen Freinamen Gefitinib, Erlotinib sowie Cetuximab erhältlichen Substanzen, welche spezifisch an den häufig in den Lungenkrebserkrankungen involvierten Rezeptor des Proliferationsfaktors EGF (*Epidermal Growth Factor),* nämlich den sogenannten EGF-Rezeptor bzw. EGFR, binden bzw. diesen inaktivieren.

Der EGF-Rezeptor (*Epidermal* *Growth* Factor-Rezeptor) ist ein Mitglied der sogenannten ErbB-Familie mit einer Unterfamilie von vier engverwandten Rezeptor-Tyrosinkinasen. Der EGF-Rezeptor wird oftmals synonym auch als HER1, EGFR1 bzw. ErbB-1 bezeichnet.

Bei dem EGF-Rezeptor handelt es sich um einen Transmembranrezeptor mit intrinsischer Tyrosinkinase-Aktivität, der in allen Zellarten vorkommt. Der Rezeptor weist einen Membrandurchgang und im zytoplasmatischen Teil eine Kinase-Domäne mit ATP-Bindungsstelle auf. Der EGF-Rezeptor gehört zu den Rezeptoren für Wachstumsfaktoren.

In nichtmalignen Zellen wird der Rezeptor nach Bindung seines Liganden (EGF) durch Dimerisierung und Phosphorylierung aktiviert und vermittelt in der Folge Wachstums- und Überlebenssignale ins Innere der Zelle. Die Aktivierung des Rezeptors führt letztendlich zu einer Stimulation des Zellwachstums und zu einer Verhinderung von Apoptose bzw. des programmierten Zelltodes. Der EGF-Rezeptor unterstützt die Proliferation und das zelluläre Überleben.

Eine Überexpression und/oder bestimmte Mutationen im EGF-Rezeptor - wie sie mitunter in Tumorzellen beobachtet werden können - vermitteln jedoch eine dauerhafte bzw. übermäßige Aktivierung des Rezeptors, was mit einem unerwünscht hohen Zellwachstum, einer übermäßigen Zellteilung und somit einer Tumorentstehung bzw. einem Tumorwachstum einhergeht. Für maligne Zellen ist eine stete Vermittlung von Wachstumssignalen von Vorteil, da sie die beschleunigte Proliferation und das Überleben der malignen Zellen bewirken bzw. unterstützen. Tumorzellen, die eine Überexpression bzw. aktivierende Mutationen in bezug auf den EGF-Rezeptor aufweisen, sind sogar von der dauerhaften bzw. übermäßigen Aktivierung des EGF-Rezeptors bezüglich ihrer Proliferation und ihres Überlebens abhängig. Der EGF-Rezeptor wird somit in verschiedenen Tumorarten hochreguliert bzw. in mutierter Form vorgefunden, was dazu führt, daß die betreffenden Tumorzellen unkontrolliert wachsen und sich vermehren. Die zuvor genannten Wirksubstanzen zielen darauf ab, das onkogene Signal des EGF-Rezeptors zu blockieren und somit das Tumorwachstum zu unterbinden bzw. zu verlangsamen.

Denn der EGF-Rezeptor kann im unmittelbaren Zusammenhang mit einer Tumor- bzw. Krebserkrankung, insbesondere einem Lungen- bzw. Bronchialkarzinom, wie dem kleinzelligen Lungenkarzinom, stehen, insbesondere da der EGF-Rezeptor in seiner mutierten Form zu einem unkontrollierten Wachstum und einer Vermehrung von Tumorzellen führt. Eine spezifische Blockierung bzw. Inaktivierung des insbesondere mutierten EGF-Rezeptors kann somit zu einer Eingrenzung bzw. Unterbindung des Wachstums von Tumorzellen führen.

Im Rahmen der vorliegenden Erfindung ist von Bedeutung, daß durch die zweckgerichtete Inhibition des EGF-Rezeptors die Aktivierung des Rezeptors vermindert bzw. gehemmt werden kann. Über 80 % der Mutationen des EGF-Rezeptors bei Patienten mit kleinzelligem Lungenkarzinom bzw. NSCLC basieren auf verschiedenen Deletionen in Exon 19 des EGF-Rezeptors sowie auf einer Punktmutation in Exon 21, nämlich der sogenannten L858R-Mutation (d. h. Austausch der Aminosäure Leucin L an Position 858 in der Aminosäuresequenz des EGF-Rezeptors durch die Aminosäure Arginin R). Patienten mit einem Lungentumor, die eine dieser Veränderungen aufweisen, eignen sich besonders für eine Therapie mit EGF-Rezeptor-Inhibitoren. Insbesondere verfügen die Medikamente bzw. Substanzen Gefitinib und Erlotinib über eine hohe Wirkspezifität in bezug auf EGF-Rezeptoren, welche derartige Mutationen aufweisen. Die Therapie mit spezifischen Inhibitoren des EGF-Rezeptors, insbesondere in bezug auf seine mutierte Form, ist im allgemeinen gut verträglich und weist auch eine gewisse Wirksamkeit auf. Aufgrund der hohen Spezifität werden selektiv die mutationtragenden Rezeptoren inhibiert, was Nebenwirkungen verringert und den Therapieeffekt vergrößert.

Die meisten Patienten entwickeln nach einer gewissen Zeit eine sogenannte Sekundärmutation, die zusätzlich zu der bereits vorhandenen Mutation auftritt und zu einer Resistenz gegenüber Erlotinib und Gefitinib führt. In etwa 65 % dieser Fälle findet man eine Mutation in Exon 20 des EGF-Rezeptors vor, wobei es sich hierbei um eine T790M-Mutation (d. h. Austausch der Aminosäure Threonin T durch Methionin M an Position 790 des EGF-Rezeptors) handelt. Für solche Patienten stehen Medikamente zur Verfügung, deren Wirkmechanismus und Spezifität sich von denjenigen Medikamenten der sogenannten ersten Generation, wie Erlotinib und Gefitinib, unterscheiden. Die Inhibitoren der sogenannten zweiten Generation binden insbesondere irreversibel an den Rezeptor und nicht reversibel, wie es bei den in Rede stehenden Medikamenten der ersten Generation der Fall ist. Patienten mit einem kleinzelligen Lungenkarzinom, die aufgrund der Sekundärmutation, insbesondere der T790M-Mutation, nicht mehr auf Medikamente der ersten Generation ansprechen, können folglich mit einem EGF-Rezeptor-Inhibitor der zweiten Generation weiterbehandelt werden. Diese Inhibitoren sind ebenfalls hochspezifisch und wirksam, so daß das Wachstum und Überleben der Tumorzellen verlangsamt oder verhindert werden kann.

Vor diesem technischen bzw. medizinischen Hintergrund kommt somit einer schnellen, einfach zu handhabenden und zu exakten Ergebnissen führenden Mutationsanalyse in bezug auf den EGF-Rezeptor bei Patienten mit einem Lungenkarzinom eine herausragende Bedeutung zu, insbesondere auch vor dem Hintergrund, den Therapieablauf im Hinblick auf den spezifischen Mutationsbefund abzustimmen bzw. zu optimieren.

Insbesondere ist es zur Gewährleistung eines optimalen therapeutischen Ansatzes mittels einer hocheffektiven und individualisierten Medizin notwendig, das Tumorgewebe auf den Status des EGF-Rezeptors, insbesondere im Hinblick auf etwaige vorliegende Mutationen, insbesondere wie zuvor angeführt, zu untersuchen. Auf dieser Basis kann bei den jeweiligen Patienten in Abhängigkeit von ihrem Mutationsstatus eine Behandlung mit den entsprechenden EGF-Rezeptor-Inhibitoren durchgeführt werden.

Auf Basis einer aussagekräftigen Mutationsanalyse in bezug auf den EGF-Rezeptor kann somit eine diesbezügliche zielgerichtete Therapie mit den jeweiligen Medikamenten realisiert werden.

Im Stand der Technik stehen zur Detektion von Mutationen in genomischer DNA aus Tumorgewebe verschiedene molekularbiologische Methoden bzw. Ansätze zur Verfügung. Standardgemäß wird zum Beispiel die Sequenzierung nach Sanger eingesetzt. Dieses Verfahren weist jedoch den Nachteil auf, daß Mutationen nur nachgewiesen werden können, wenn die sie tragende DNA mindestens mit einem Anteil von 20 % bis 25 % in der zu analysierenden Probe, bezogen auf den Gesamt-DNA-Gehalt der Probe, vorliegt. Zudem ist der Zeitaufwand für die Durchführung und Auswertung relativ hoch, da die Versuchsdurchführung mehrere Stunden betragen kann.

Ein weiteres Verfahren des Standes der Technik zur Analyse von Mutationen besteht in der sogenannten Polymerase-Kettenreaktion bzw. *Polymerase Chain Reaction* bzw. PCR, wie beispielsweise der sogenannten Real-Time-PCR bzw. RT-PCR. Durch den Einsatz dieser Methode kann die Analysedauer verringert werden. Zudem ist die Durchführung relativ kostengünstig und die Sensitivität gegenüber der zu detektierenden bzw. zu analysierenden Mutation bereits erhöht. Dennoch sind die mittels herkömmlicher PCR erhaltenen Ergebnisse nicht immer zufriedenstellend, insbesondere sofern die Probe nur äußerst geringe Mengen an Mutationsmaterial aufweist. Die herkömmliche PCR weist im Ergebnis nur eine geringe Sensitivität auf.

In Nagai Y. et al. (2005), Cancer Res. 65(16):7276-7282 wird ein PCR-basiertes Nachweisverfahren für die Detektion von EGFR-Mutationen bei NSCLC-Lungenkrebs offenbart, wobei die Primer mit einer mutationsspezifischen LNA-Sensorsonde kombiniert werden, die spezifisch für die Mutante ist. Das Wildtypgen wird durch ein Blockierungsmittel ("PNA clamp primer") maskiert, welches in der Amplifikationsphase zu einer bevorzugten Vervielfältigung der mutierten Ziel-DNA führt, und in der Detektionsphase die Fehlpaarung der Sensorsonde an die Wildtypsequenz unterbindet.

Vor diesem technischen Hintergrund besteht nunmehr die Aufgabe der vorliegenden Erfindung darin, ein Verfahren zum Nachweis bzw. zur Detektion von Genveränderungen, insbesondere Mutationen, bereitzustellen, welches die zuvor geschilderten Nachteile des Standes der Technik zumindest teilweise vermeidet oder aber wenigstens abschwächt.

Insbesondere soll ein Verfahren bereitgestellt werden, welches eine sehr hohe Sensitivität aufweist, d. h. bereits bei sehr geringen Mengen an Mutationsmaterial in einer Probe bzw. einem zu analysierenden Material zu aussagekräftigen Ergebnissen führt.

Zudem soll das erfindungsgemäße Verfahren an einer Vielzahl an verschiedenartigen Proben bzw. Patientenmaterialien, wie beispielsweise Blutproben, Lymphflüssigkeit, Zellen, aufgereinigter DNA oder dergleichen, eingesetzt werden können.

Das erfindungsgemäße Verfahren soll zudem eine fundierte Aussage im Hinblick auf einen sozusagen mutationsabhängigen bzw. -spezifischen therapeutischen Ansatz ermöglichen, um auf diese Weise das zugrundeliegende Behandlungsschema, insbesondere im Hinblick auf die Auswahl spezieller Medikamente, zu optimieren.

Insbesondere soll das erfindungsgemäß vorgeschlagene Verfahren zur Detektion bzw. Analyse von Mutationen in Proteinen, insbesondere dem EGF-Rezeptor, geeignet sein, wobei die Mutation bzw. das Protein mit der Mutation im Zusammenhang mit der Entwicklung bzw. dem Auftreten von Lungenkarzinomen, insbesondere des kleinzelligen Lungenkarzinoms bzw. NSCLC, steht.

Zur Lösung der zuvor geschilderten Aufgabenstellung schlägt die vorliegende Erfindung gemäß einem ersten Erfindungsaspekt ein Verfahren zum Nachweis bzw. zur Detektion mindestens einer Genveränderung, insbesondere Mutation, in einem Gen vor, vorzugsweise in einem für ein mit einer Tumor- und/oder Krebserkrankung in Verbindung stehenden Protein kodierenden Gen, insbesondere wobei das die Genveränderung aufweisende Gen zusammen mit weiteren, für das Protein kodierenden, jedoch keine Genveränderung aufweisenden Genen (Wildtypgenen) vorliegen kann, gemäß Anspruch 1 vor. Weitere, vorteilhafte Ausgestaltungen dieses Erfindungsaspekts sind Gegenstand der diesbezüglichen Unteransprüche.

Weiterhin ist Gegenstand der vorliegenden Erfindung gemäß einem zweiten Erfindungsaspekt eine Zusammensetzung gemäß Anspruch 9, welche sich insbesondere zur Verwendung im Rahmen einer asymmetrischen Polymerase-Kettenreaktion, vorzugsweise zum Nachweis bzw. zur Detektion mindestens einer Genveränderung, insbesondere Mutation, eignet.

Gegenstand der vorliegenden Erfindung gemäß einem dritten Erfindungsaspekt ist zudem die Verwendung der Zusammensetzung nach der Erfindung zum Nachweis bzw. zur Detektion mindestens einer Genveränderung, insbesondere Mutation, gemäß Anspruch 10.

Es versteht sich von selbst, daß Ausgestaltungen, Ausführungsformen, Vorteile und dergleichen, welche nachfolgend zu Zwecken der Vermeidung von Wiederholungen nur zu einem Erfindungsaspekt aufgeführt sind, selbstverständlich auch in bezug auf die übrigen Erfindungsaspekte entsprechend gelten.

Ein erster Gegenstand der vorliegenden Erfindung - gemäß einem **ersten** Aspekt der vorliegenden Erfindung - ist somit ein Verfahren zum Nachweis bzw. zur Detektion mindestens einer Genveränderung, insbesondere Mutation, in einem Gen, vorzugsweise in einem für ein mit einer Tumor- und/oder Krebserkrankung in Verbindung stehenden Protein kodierenden Gen, insbesondere wobei das die Genveränderung aufweisende Gen (= mutiertes Gen) bzw. das die Genveränderung aufweisende Allel (= mutiertes Allel) - synonym jeweils auch als Mutationsgen bzw. Mutationsallel bezeichnet - zusammen mit weiteren, für das Protein kodierenden, jedoch keine Genveränderung aufweisenden Genen bzw. Allelen (= Wildtypgenen bzw. Wildtypallelen) vorliegt, wobei sich das erfindungsgemäße Verfahren dadurch auszeichnet, daß das Verfahren mittels asymmetrischer Polymerase-Kettenreaktion (*Polymerase Chain Reaction* bzw. PCR) unter kombinierter Verwendung mindestens einer detektierbaren mutationspezifischen Hybridisierungssonde (Sensorsonde) einerseits und mindestens eines die Anbindung der Sensorsonde an die Wildtypgene bzw. Wildtypallele inhibierenden wildtypspezifischen Blockierungsmittel (= *Clamp)* durchgeführt wird, insbesondere so daß eine selektive Verstärkung und/oder Amplifizierung der Detektion in bezug auf einen die Genveränderung aufweisenden Genabschnitt bzw. Abschnitt, insbesondere DNA-Abschnitt, des Mutationsgens bzw. Mutationsallels erfolgt.

Die grundlegende Idee der vorliegenden Erfindung ist somit darin zu sehen, daß aufgrund der spezifischen Konzeption des erfindungsgemäßen Verfahrens Genveränderungen bzw. Mutationen in einer Probe bzw. einem Ensemble von Genen mit sehr hoher Sensitivität erfaßt werden können, so daß bereits kleinste Mengen an mutierter DNA (= mt-DNA), insbesondere in einem Gemisch bzw. Ensemble mit Wildtyp-DNA bzw. keine Mutation aufweisender DNA (= wt-DNA), analysiert werden können. Im Rahmen der vorliegenden Erfindung ist es somit gelungen, bereits geringe Mengen bzw. Gehalte an DNA, welche die Genveränderung bzw. Mutation aufweisen, in einer Probe mit hoher Zuverlässigkeit zu analysieren und zwar insbesondere auch insofern, als eine konkrete Bestimmung bzw. ein spezieller Nachweis von bestimmten Mutationen erfolgen kann.

In diesem Zusammenhang ist es erfindungsgemäß möglich, bereits geringe Mengen an mutierter DNA ab einem Gehalt von etwa 0,0025 % in einem Gemisch mit sonstiger DNA bzw. nichtmutierter DNA bzw. Wildtyp-DNA, bezogen auf den DNA-Gehalt, zu detektieren.

In diesem Zusammenhang besteht die grundlegende Idee der vorliegenden Erfindung darin, die Effektivität bzw. Sensitivität des Verfahrens dadurch gezielt zu erhöhen bzw. zu verbessern, daß in zweckgerichteter Weise eine asymmetrische Polymerase-Kettenreaktion durchgeführt wird, bei welcher vorrangig bzw. selektiv eine Vermehrung bzw. Amplifizierung des DNA-Einzelstranges des Mutationsgens erfolgt, welcher die Mutation aufweist und an welchem die zur eigentlichen Detektion im Sinne einer meßtechnischen Erfassung dienende Sensorsonde spezifisch bindet. Dies kann beispielsweise, wie nachfolgend noch ausführlich angeführt, dadurch realisiert werden, daß unterschiedliche Mengen an Primer, welche synonym auch als (PCR-)Startermoleküle bezeichnet werden, eingesetzt werden, wobei im Rahmen der vorliegenden Erfindung insbesondere die Menge desjenigen Primer erhöht wird, welcher an dem sogenannten Sondenstrang mit der Mutation bindet. Hierdurch wird erfindungsgemäß erreicht, daß die Menge an DNA-Einzelsträngen mit der Mutation, in deren Bereich die Sensorsonde spezifisch bindet, im Vergleich zu den übrigen DNA-Einzelsträngen erhöht wird, so daß in der Probe der DNA-Strang, an welchem die Sensorsonde spezifisch hybridisiert, gewissermaßen überrepräsentiert ist und somit aufgrund der statistisch häufigeren Bindung der Sensorsonde ein verstärktes Sensorsignal resultiert.

Zudem ist es im Rahmen der vorliegenden Erfindung in völlig überraschender Weise gelungen, die Sensitivität noch dadurch weiter zu erhöhen, daß zum einen eine mutationsspezifische Hybridisierungssonde bzw. Sensorsonde eingesetzt wird, welche eine erhöhte Bindungsaffinität bzw. Spezifität bzw. Selektivität zu der Mutationsstelle bzw. dem Mutationsbereich in dem mutierten DNA-Strang bzw. DNA-Einzelstrang im Vergleich zu dem hierzu korrespondierenden bzw. entsprechenden wildtypischen DNA-Einzelstrang - d. h. zu dem originären Einzelstrang ohne Mutation - aufweist. Zum anderen wird im Rahmen des erfindungsgemäßen Verfahrens die asymmetrische Polymerase-Kettenreaktion zudem in Gegenwart eines speziellen Blockierungsmittels bzw. Blockers durchgeführt, welches bzw. welcher nämlich spezifisch, d. h. mit erhöhter Affinität, an den DNA-Einzelstrang ohne Mutation bzw. den wildtypischen DNA-Einzelstrang an dem dem Mutationsbereich entsprechenden bzw. hierzu korrespondierenden Abschnitt bindet und somit eine unspezifische Anbindung der Sensorsonde an diesem Bereich der nichtmutierten DNA verhindert bzw. verringert.

Auf Basis dieser sozusagen ternären Kombination von Maßnahmen ist es im Rahmen der vorliegenden Erfindung in völlig überraschender Weise gelungen, ein Verfahren zur Detektion von Mutationen bzw. Genveränderungen bereitzustellen, welches zu einer deutlichen Verstärkung speziell des auf die zu untersuchende Mutation zurückgehenden Meßsignals im Sinne einer Diskriminierung bzw. Verstärkung gegenüber den sonstigen Signalen in der Probe führt, so daß auf Basis des erfindungsgemäßen Verfahrens bereits sehr geringe Mengen an mutierter DNA in einer Probe bzw. Ausgangsmaterial analysiert werden können. Aufgrund des hochsensitiven Verfahrens ist es im Rahmen der vorliegenden Erfindung möglich, auf Proben zurückzugreifen, welche nur geringe Mengen an auf Tumorzellen zurückgehender DNA aufweisen. Somit können im Rahmen der vorliegenden Erfindung bereits kleinste Anteile mutierter DNA nachgewiesen werden, ohne daß hierzu aufwendige und aus medizinischer Sicht mitunter problematische Biopsien durchgeführt werden müßten. Grundsätzlich können erfindungsgemäß aber auch Zellproben bzw. Zellmaterial, z. B. Tumorzellen als solche dem erfindungsgemäßen Verfahren zugrundegelegt werden

Auf Basis des erfindungsgemäßen Verfahrens mit der aussagekräftigen Detektion bzw. Analyse von Mutationen ist es möglich, in gezielter Weise eine Aussage über den Krankheitszustand bzw. eine Verlaufskontrolle, insbesondere in bezug auf Arzneimittelwirkungen oder dergleichen, zu ermöglichen, wobei zudem auf Basis der konkreten Mutationsanalyse diesbezüglich optimierte Tumortherapien, beispielsweise unter Verwendung spezifischer Inhibitoren bzw. zielgerichteter Medikamente, durchgeführt werden können.

Wie zuvor angeführt, ist es im Rahmen der vorliegenden Erfindung möglich, daß das mutierte Gen (synonym auch als Mutationsgen bezeichnet) bzw. die mutierten Gene (synonym auch als Mutationsgene bezeichnet) gewissermaßen in einem Ensemble bzw. einer Probe gemeinsam mit einem Wildtypgen bzw. mit Wildtypgenen vorliegen, was beispielsweise dann der Fall ist, wenn in einer bereitgestellten (Ausgangs-)Probe sowohl Tumorzellen als auch nichtentartete Zellen vorliegen.

Im Rahmen der vorliegenden Erfindung ist es auch möglich, daß die entsprechenden Gene Mutationsallele und Wildtypallele aufweisen bzw. aus diesen bestehen. So kann beispielsweise das Mutationsgen im Sinne einer homozygoten Ausprägung zwei Mutationsallele aufweisen, d. h. beide Allele des Gens sind Träger der entsprechenden Genveränderung bzw. Mutation. Gleichermaßen kann es möglich sein, daß das Mutationsgen heterozygot ausgebildet ist, wobei in diesem Fall ein Allel, nämlich das Mutationsallel, mit der Genveränderung bzw. Mutation und - hierzu korrespondierend - ein Wildtypallel ohne Mutation in bezug auf das Mutationsgen vorliegt. In bezug auf das Wildtypgen, welches insbesondere aus gesunden bzw. nichtmalignen Zellen stammt, liegen beide Allele insbesondere in der Wildtyp-Form bzw. als Wildtypallele vor. Insofern können im nachfolgenden die verwendeten Begriffe "Mutationsgen" bzw. "Wildtypgen" sich insbesondere auch auf die entsprechenden Allele, wie zuvor definiert, beziehen. Das erfindungsgemäße Verfahren eignet sich somit auch zum Nachweis bzw. zur Detektion von Mutationen in den jeweiligen Allelen eines Gens.

Was die vorliegende Erfindung weiter anbelangt, so kann es sich bei der Tumor- oder Krebserkrankung um ein Lungenkarzinom, insbesondere um ein nichtkleinzelliges Lungenkarzinom (*Non Small Cell Lung Cancer* bzw. NSCLC) und/oder um ein kleinzelliges Lungenkarzinom (*Small Cell Lung Cancer* bzw. SCLC), vorzugsweise um ein nichtkleinzelliges Lungenkarzinom (NSCLC), handeln.

Mit anderen Worten fokussiert die vorliegende Erfindung darauf, Mutationen nachzuweisen bzw. zu detektieren, die im Zusammenhang mit den vorgenannten Krebserkrankungen, insbesondere im Zusammenhang mit einem nichtkleinzelligen Lungenkarzinom, stehen bzw. diese Erkrankungen, beispielsweise durch eine abnorme Funktion des resultierenden Genproduktes spielsweise durch eine abnorme Funktion des resultierenden Genproduktes des Mutationsgens, hervorrufen. Die vorliegende Erfindung ist jedoch nicht auf die vorgenannten Erkrankungen beschränkt. Vielmehr kann das erfindungsgemäße Verfahren universell zur Analyse bzw. Detektion von insbesondere bekannten Mutationen bzw. Genveränderungen, die im Zusammenhang mit Erkrankungen des Organismus als solchen stehen, eingesetzt werden. Dies kann beispielsweise über eine spezifische Anpassung der mutationsspezifischen Hybridisierungssonden bzw. der Sensorsonden, der Primer und/oder des Blockierungsmittels realisiert werden, so daß das erfindungsgemäße Verfahren gewissermaßen für eine Vielzahl spezieller Erkrankungen maßgeschneidert werden kann.

Bei dem in Rede stehenden Protein, dessen Veränderung über die Erfassung bzw. Analyse der Mutation des für das Protein kodierenden Gens bestimmt werden kann, handelt es sich gemäß einer bevorzugten erfindungsgemäßen Ausführungsform um ein insbesondere humanes und/oder ein das Zellwachstum und/oder die Proliferation von Zellen regulierendes und/oder induzierendes Protein. Bei dem Protein kann es sich zudem um einen insbesondere transmembranen Rezeptor für Wachstumsfaktoren, insbesondere mit intrinsischer Tyrosinkinaseaktivität, handeln. Bevorzugt handelt es sich bei dem in Rede stehenden Protein, welches im Zusammenhang mit einer Tumor- oder Krebserkrankung steht, um den Epidermalen-Wachstumsfaktor-Rezeptor (*Epidermal-Growth-Factor-*Rezeptor bzw. EGF-Rezeptor), insbesondere wie zuvor beschrieben. Insbesondere handelt es sich im Rahmen der vorliegenden Erfindung um den humanen EGF-Rezeptor.

Die vorliegende Erfindung umfaßt gleichermaßen auch strukturidentische bzw. strukturähnliche Proteine mit Wirkidentität bzw. Wirkähnlichkeit zu dem zuvor genannten EGF-Rezeptor. Der Begriff "EGF-Rezeptor", wie er im Rahmen der vorliegenden Erfindung verwendet wird, umfaßt insbesondere auch funktionsidentische bzw. funktionsähnliche Abwandlungen und auch nicht unmittelbar mit der Krebs- bzw. Tumorerkrankung in Verbindung stehende Mutationsformen des EGF-Rezeptors an sich. Insbesondere umfaßt der Begriff "EGF-Rezeptor" auch insbesondere zumindest im wesentlichen funktionsähnliche bzw. funktionsidentische Isoformen und/oder Vorläufer bzw. Präkursoren. Gemäß einer erfindungsgemäß bevorzugten Ausführungsform handelt es sich bei dem in Rede stehenden Protein um den EGF-Rezeptor gemäß dem Locus bzw. gemäß der Referenznummer NP_005219.2 und/oder insbesondere gemäß Sequenzprotokoll I und/oder Sequenzprotokoll II (Sequence Listing). Im allgemeinen umfaßt die vorliegende Erfindung auch funktionale Analoga und/oder Isoformen des in Rede stehenden EGF-Rezeptors bzw. solche Formen des EGF-Rezeptors bzw. Proteine im allgemeinen, welche eine Übereinstimmung in der Aminosäuresequenz von mindestens 90 %, insbesondere mindestens 95 %, vorzugsweise mindestens 98 %, bevorzugt mindestens 99 %, bezogen auf die Aminosäuresequenz des EGF-Rezeptors gemäß Sequenzprotokoll I und/oder gemäß Sequenzprotokoll II, aufweisen.

Das Sequenzprotokoll I und das Sequenzprotokoll II (Sequence Listing) beziehen sich auf die Aminosäuresequenz des EGF-Rezepors. Dabei ist das Sequenzprotokoll I zu dem Sequenzprotokoll II in bezug auf die Aminosäuresequenz synonym bzw. inhaltsgleich. Der wesentliche Unterschied zwischen den Sequenzprotokollen besteht darin, daß das Sequenzprotokoll I auf Basis einer wissenschaftlich standardisierten Angabe bzw. Darstellung beruht, während das Sequenzprotokoll II auf Basis einer patentrechtlich standardisierten Angabe bzw. Darstellung unter Verwendung der Software PatentIn Version 3.3 erstellt wurde. Hierbei handelt es sich also in bezug auf die Aminosäuresequenz um einen rein formal darstellungsgemäßen, nicht aber um einen inhaltlichen Unterschied.

Die im Rahmen des erfindungsgemäßen Verfahrens zu untersuchende bzw. zu analysierende Genveränderung, insbesondere Mutation, kann somit zu einer erhöhten und/oder übermäßigen Aktivität des von dem entsprechenden Gen kodierten Proteins führen und/oder hiermit im Zusammenhang stehen. Somit kann die Genveränderung bzw. Mutation zu einem erhöhten bzw. übermäßigen Zellwachstum und/oder zu einer erhöhten bzw. übermäßigen Zellproliferation führen bzw. hiermit im Zusammenhang stehen. Die zu untersuchende Genveränderung kann zu einer pathologischen bzw. von der physiologischen Norm abweichenden Aktivität des von dem entsprechenden Gen kodierten Proteins, insbesondere des EGF-Rezeptors, führen. Mit anderen Worten bewirkt die Genveränderung, insbesondere Mutation, eine Änderung in dem entsprechenden Genprodukt, welche im Zusammenhang mit der Tumor- bzw. Krebserkrankung steht. Bei der Erkrankung, welche im Zusammenhang mit der Genveränderung steht, handelt es sich insbesondere, wie zuvor angeführt, um ein Bronchialkarzinom, insbesondere um NSCLC.

Bei der nachzuweisenden bzw. zu analysierenden Genveränderung kann es sich beispielsweise um eine Punktmutation und/oder eine Rastermutation und/oder um eine Deletion handeln. Im Rahmen einer bevorzugten Ausführungsform des erfindungsgemäßen Verfahrens handelt es sich bei der Genveränderung um eine Punktmutation, insbesondere bei welcher singuläre Basen an spezifischen Stellen des Gens bzw. DNA-Stranges ausgetauscht bzw. verändert sind.

Beispielsweise kann die Genveränderung derart sein, daß diese zu mindestens einer Deletion in Exon 19 des EGF-Rezeptors führt und/oder hiermit im Zusammenhang steht. Gemäß einer bevorzugten Ausführungsform handelt es sich bei der Genveränderung um eine Mutation, insbesondere Punktmutation, welche zu einem Austausch mindestens einer Aminosäure in Exon 20 und/oder Exon 21 des EGF-Rezeptors als entsprechendes Genprodukt führt bzw. hiermit im Zusammenhang steht. Diesbezüglich kann die Genveränderung zu einem Austausch von Serin an Position 768 des EGF-Rezeptors insbesondere durch Isoleucin (S768I) führen bzw. hiermit im Zusammenhang stehen. Im Rahmen der vorliegenden Erfindung handelt es sich bei der Genveränderung jedoch vorzugsweise um eine solche, welche zu einem Austausch von Threonin an Position 790 des EGF-Rezeptors insbesondere durch Methionin (T790M) führt bzw. hiermit im Zusammenhang steht bzw. um eine solche Genveränderung, welche zu einem Austausch von Leucin an Position 858 des EGF-Rezeptors insbesondere durch Arginin (L858R) führt bzw. hiermit im Zusammenhang steht.

Die vorliegende Erfindung zielt somit vorrangig auf eine Detektion bzw. Analyse bzw. auf eine Untersuchung des Vorhandenseins der Punktmutationen L858R und/oder T790M ab. Bei T790M handelt es sich um eine Mutation in Exon 20 des EGF-Rezeptors, während es sich bei L858R um eine Mutation in Exon 21 des EGF-Rezeptors handelt. Wie zuvor dargestellt, handelt es sich bei den in Rede stehenden Mutationen L858R und T790M des EGF-Rezeptors um spezifische Mutationen, welche im Zusammenhang mit der Induktion bzw. Ausbildung bzw. Progression von Lungenkarzinomen, insbesondere dem nichtkleinzelligen Lungenkarzinom (NSCLC), stehen und diesbezüglich eine Zielstruktur bzw. Target für spezifische Arzneimittel, wie zuvor angeführt, darstellen.

Die vorliegende Erfindung ist jedoch nicht auf die Detektion bzw. Analyse der vorgenannten speziellen Mutationen des EGF-Rezeptors beschränkt. Vielmehr kann das erfindungsgemäße Verfahren in bezug auf eine Vielzahl an Mutationen eingesetzt werden, insbesondere sofern die zu detektierenden bzw. zu analysierenden Mutationen im Zusammenhang mit der Ausbildung bzw. dem Vorhandensein einer Tumor- bzw. Krebserkrankung, insbesondere einem Bronchialkarzinom, wie dem kleinzelligen Lungenkarzinom, stehen. Der Fachmann ist jederzeit in der Lage, die für die jeweils zu untersuchenden Mutationen spezifischen Komponenten, wie Primer, die nachfolgend noch beschriebenen Sonden und/oder das Blockierungsmittel, auszuwählen und im Rahmen des erfindungsgemäßen Verfahrens einzusetzen.

Wie zuvor angeführt, kann es erfindungsgemäß vorgesehen sein, daß das Mutationsgen bzw. die Mutationsgene einerseits und das Wildtypgen bzw. die Wildtypgene andererseits in einer Probe und/oder einem Ensemble vorliegen, insbesondere wobei die bereitgestellte Probe und/oder das Ensemble von einem Patienten stammt und/oder insbesondere wobei die Probe und/oder das Ensemble von einem Körpermaterial, insbesondere einer Körperflüssigkeit, vorzugsweise Blut und/oder Gewebsflüssigkeit und/oder Lymphflüssigkeit und/oder Urin mit insbesondere zellularen Bestandteilen, stammt bzw. hieraus gewonnen werden kann. Zudem kann auch bereitgestelltes Zellmaterial bzw. Tumormaterial eingesetzt werden, beispielsweise auf Basis einer Biopsie.

Im allgemeinen kann die dem erfindungsgemäßen Verfahren zugrundeliegende Probe derart sein, daß sie sowohl gesunde bzw. nichtmaligne Zellen mit intaktem EGF-Rezeptor sowie den diesbezüglich kodierenden Wildtypgenen als auch Tumorzellen mit gegebenenfalls mutiertem EGF-Rezeptor und den diesbezüglich zugehörigen Mutationsgenen bzw. Mutationsallelen aufweist. Beispielsweise kann es sich bei der Probe um eine solche handeln, welche neben normalen bzw. nicht entarteten Zellen auch zirkulierende Tumorzellen aufweist.

Die Probe bzw. das Ausgangsmaterial kann zudem vor Durchführung des erfindungsgemäßen Verfahrens weiter aufgereinigt werden bzw. die DNA aus der Probe zur weiteren Verwendung isoliert bzw. aufkonzentriert bzw. aufgereinigt werden. Die diesbezüglichen Methoden sind dem Fachmann hinlänglich bekannt, so daß es hierzu keiner weiteren Ausführungen bedarf.

Im Rahmen der vorliegenden Erfindung ist es dabei möglich, daß Genveränderungen bzw. Mutationen auf Basis einer Probe bestimmt werden können, welche weniger als 1.000, insbesondere weniger als 500, vorzugsweise weniger als 200, besonders bevorzugt weniger als 100 Tumorzellen, pro ml Probe aufweisen. Das erfindungsgemäße Verfahren kann auf Basis von Proben bzw. Ausgangsmaterialien mit DNA durchgeführt werden, welche weniger als 1 %, insbesondere weniger als 0,1 %, vorzugsweise weniger als 0,01 %, bevorzugt weniger als 0,001 % Tumorzellen, bezogen auf den Gesamtzellgehalt der Probe, aufweisen.

Das erfindungsgemäße Verfahren kann in bezug auf eine Probe bzw. ein Ausgangsmaterial angewendet werden kann, welche(s) weniger als 1 % mutierte DNA, insbesondere etwa 0,5 %, vorzugsweise etwa 0,05 %, bevorzugt etwa 0,005 %, besonders bevorzugt etwa 0,0005 % mutierte DNA, bezogen auf die Gesamt-DNA in der Probe bzw. in dem Ausgangsmaterial, aufweist. Somit handelt es sich bei dem erfindungsgemäßen Verfahren um ein hochsensitives und effizientes Verfahren, bei dem selbst geringste Spuren an DNA hinsichtlich des Vorliegens von Mutationen aussagekräftig analysiert werden können.

Im Rahmen der vorliegenden Erfindung kann es zudem vorgesehen sein, daß die asymmetrische PCR in Gegenwart von Primern, insbesondere in Form von Oligonukleotiden, durchgeführt wird.

In diesem Zusammenhang können die Primer ausgewählt werden derart, daß insbesondere im Rahmen der PCR eine Amplifizierung des die Genveränderung aufweisenden Genabschnitts des Mutationsgens bzw. Mutationsallels erfolgt und/oder daß eine Amplifizierung des zu dem die Genveränderung aufweisenden Genabschnitt des Mutationsgens korrespondierenden Genabschnitts der Wildtypgene bzw. der Wildtypallele erfolgt. Die Formulierung "Der zu dem die Genveränderung aufweisenden Genabschnitt des Mutationsgens korrespondierende Genabschnitt des Wildtypgens" ist dabei insbesondere so zu verstehen, daß der zu dem Abschnitt mit der Mutation entsprechende Abschnitt des Wildtypgens bzw. Wildtypallele, bei welchem als solchen keine Mutation vorliegt, gemeint ist.

Durch die Auswahl der Primer ist eine gezielte Amplifizierung bzw. Vermehrung des für die Untersuchung bzw. die Genanalyse relevanten Genabschnitts bzw. des hierzu korrespondierenden Genabschnitts des Wildtypgens und somit eine Diskriminierung gegenüber den übrigen Genen bzw. der übrigen DNA in der Probe möglich. Insbesondere handelt es sich bei der zu amplifizierenden DNA um das Gen des zuvor beschriebenen EGF-Rezeptors bzw. vorzugsweise um einen Genabschnitt des für das EGF-Protein kodierenden Gens, und zwar insbesondere um denjenigen Genabschnitt, in welchem die Mutation vorliegen kann. Die konkrete Auswahl der diesbezüglich einzusetzenden Primer ist dem Fachmann an sich wohlbekannt, und der Fachmann ist jederzeit in der Lage, die entsprechenden Primer auszuwählen und einzusetzen.

Die im Rahmen der vorliegenden Erfindung eingesetzten bzw. verwendeten Primer können ausgewählt werden derart, daß eine Amplifizierung des die Deletion in Exon 19 des EGF-Rezeptors aufweisenden Genabschnitts des Mutationsgens erfolgt. Gleichermaßen kann es im Rahmen der vorliegenden Erfindung vorgesehen sein, daß die Primer ausgewählt werden derart, daß eine Amplifizierung des den Austausch von Serin an Position 768 des EGF-Rezeptors insbesondere durch Isoleucin (S768I) aufweisenden Genabschnitts des Mutationsgens erfolgt. Gemäß einer erfindungsgemäßen besonders bevorzugten Ausführungsform können die Primer ausgewählt werden derart, daß eine Amplifizierung des den Austausch von Threonin an Position 790 des EGF-Rezeptors insbesondere durch Methionin (T790M) aufweisenden Genabschnitts des Mutationsgens erfolgt und/oder daß eine Amplifizierung des den Austausch von Leucin an Position 858 des EGF-Rezeptors insbesondere durch Arginin (L858R) aufweisenden Genabschnitts des Mutationsgens erfolgt.

Zudem kann es diesbezüglich gegebenenfalls möglich sein, daß gleichzeitig eine Amplifizierung des zu dem die Genveränderung aufweisenden Genabschnitt des Mutationsgens korrespondierenden Genabschnitts der Wildtypgene erfolgen kann, insbesondere aufgrund der Eigenschaften der Primer als solche.

Was die zu amplifizierenden Genabschnitte anbelangt, so sollte die diesbezügliche Größe bzw. die Anzahl an Basenpaaren ausgewählt werden derart, daß die Sensorsonde sowie die gegebenenfalls gleichermaßen an den Genabschnitt bindende und nachfolgend noch beschriebene Ankersonde an den entsprechenden DNA-Einzelsträngen der jeweiligen Genabschnitte zu binden imstande sind.

Im Rahmen der vorliegenden Erfindung kann es vorgesehen sein, daß ein erster Primer und ein hiervon verschiedener zweiter Primer eingesetzt werden. In diesem Zusammenhang sollte der erste Primer zumindest im wesentlich spezifisch an den DNA-Einzelstrang des Mutationsgens (Sondenstrang) binden, an welchem die Sensorsonde zu binden bzw. zu hybridisieren imstande ist bzw. mit welchem die Sensorsonde zu interagieren imstande ist. Gleichermaßen kann der erste Primer aufgrund der Natur der Primer auch an dem hierzu korrespondierenden DNA-Einzelstrang des Wildtypgens binden. Bei dem ersten Primer handelt es sich somit gewissermaßen um einen sense-Primer. Was den zweiten Primer anbelangt, so sollte dieser zumindest im wesentlichen spezifisch an dem zum Sondenstrang komplementären DNA-Einzelstrang des Mutationsgens (Komplementärstrang) binden bzw. hiermit zu binden bzw. zu interagieren imstande sein. Bei dem zweiten Primer handelt es sich gewissermaßen um einen sogenannten *antisense*-Primer. Der zweite Primer kann zudem an dem diesbezüglich korrespondierenden DNA-Einzelstrang des Wildtypgens binden.

Im Falle der speziellen Detektion bzw. Analyse der Mutation T790M sollten der erste und zweite Primer derart ausgewählt werden, daß spezifisch der diese Mutation aufweisende Genabschnitt des Mutationsgens bzw. des korrespondierenden Wildtypgens im Rahmen der PCR amplifiziert wird, entsprechendes gilt für die Analyse bzw. Detektion der Mutation L858R.

Im Fall der Analyse bzw. Detektion der Mutation T790M kann der erste Primer in nichtbeschränkender Weise insbesondere die Basenabfolge bzw. Nukleotidsequenz GACTCCGACTCCTCCTTTATCCAATG aufweisen bzw. aus dieser Nukleotidsequenz bestehen. Im Falle des zweiten Primers kann dieser in nichtbeschränkender Weise die Nukleotidsequenz CACACAC-CAGTTGAGCAGGTA aufweisen bzw. hieraus bestehen.

Im Falle der Analyse bzw. Detektion der Mutation L858R kann der erste Primer die Nukleotidsequenz GCTCAGAGCCTGGCATGAA aufweisen bzw. hieraus bestehen; der zweite Primer kann die Nukleotidsequenz CATCCTCCCCTGCATGTGT aufweisen bzw. hieraus bestehen.

Die jeweiligen Primer umfassen insbesondere auch solche Primer, welche eine vergleichbare Spezifität bzw. Selektivität in bezug auf die jeweiligen Genabschnitte aufweisen. Dies ist dem Fachmann aber an sich bekannt, und der Fachmann ist jederzeit in der Lage, vor dem Hintergrund der Amplifizierung der entsprechenden Genabschnitte die jeweils spezifischen Primer auszuwählen. Die Primer sollten ausgewählt werden derart, daß diese selbst außerhalb des Bereichs der zu analysierenden Mutation des Mutationsgens bzw. der hierzu korrespondierenden Abschnitte des Wildtypgens bzw. den diesbezüglichen DNA-Einzelsträngen binden.

Im Rahmen der vorliegenden Erfindung ist es besonders bevorzugt, wenn die asymmetrische PCR durchgeführt wird derart, daß der DNA-Einzelstrang, an welchem die Sensorsonde (Sondenstrang) zu binden imstande ist, stärker bzw. häufiger amplifiziert wird als der hierzu komplementäre DNA-Einzelstrang (Komplementärstrang), so daß nach Amplifizierung im PCR-Ansatz mehr Kopien des Sondenstranges im Vergleich zu dem Komplementärstrang vorliegen. Dies kann im Rahmen der Erfindung gemäß einer besonders bevorzugten Ausführungsform beispielsweise dadurch geschehen, daß der erste Primer und der zweite Primer ausgewählt werden derart, daß die Menge und/oder die Konzentration des ersten Primers, insbesondere bezogen auf den PCR-Ansatz, größer ist als die Menge und/oder Konzentration des zweiten Primers, insbesondere so daß eine erhöhte und/oder verstärkte Amplifikation des Sondenstranges gegenüber dem Komplementärstrang erfolgt. Auf diese Weise kommt es zu einer verstärkten Amplifizierung des Sondenstranges gegenüber dem komplementären DNA-Einzelstrang, was dazu führt, daß der DNA-Einzelstrang, an welchem die Sensorsonde zu binden imstande ist, in einer höheren Kopienanzahl vorliegt, so daß aufgrund der größeren Anzahl an Ereignissen in bezug auf die Bindung zwischen Sensorsonde einerseits und entsprechendem DNA-Einzelstrang andererseits eine Verstärkung bzw. Amplifizierung des mutationsspezifischen Sensorsondensignals erfolgen kann.

In diesem Zusammenhang ist es im Rahmen der vorliegenden Erfindung bevorzugt, wenn das mengenmäßige Verhältnis des ersten Primers zu dem zweiten Primer (erster Primer : zweiter Primer), insbesondere in dem PCR-Ansatz, im Bereich von 1.000 : 1 bis 1,05 : 1, insbesondere 100 : 1 bis 1,5 : 1, vorzugsweise 10 : 1 bis 2 : 1, liegt.

Dementsprechend ist es bevorzugt, wenn nach erfolgter Amplifizierung die Anzahl bzw. Konzentration des Sondenstranges gegenüber dem Komplementärstrang um einen Faktor von mindestens 1,1, insbesondere 1,5, vorzugsweise 2, bevorzugt 10, besonders bevorzugt 100, erhöht ist.

Wie zuvor angeführt, wird durch das spezielle Verhältnis der Primer mit der Bevorzugung des ersten Primers erreicht, daß im Rahmen der PCR-basierten Amplifikation vorrangig derjenige DNA-Einzelstrang des Mutationsgens bzw. des Mutationsallels amplifiziert bzw. vermehrt wird, an welchem die Sensorsonde zu binden imstande ist. Auf diese Weise wird das sensorsondenspezifische Signal zusätzlich verstärkt.

Was die Sensorsonde (Reportersonde) als solche anbelangt, so handelt es sich hierbei um eine Hybridisierungssonde, welche insbesondere im Rahmen der PCR und den diesbezüglichen Amplifizierungsschritten an den DNA-Einzelstrang des Mutationsgens bzw. Mutationsallels im Bereich der zu analysierenden bzw. zu untersuchenden Mutation zu binden bzw. zu hybridisieren imstande ist, wobei die Mutation als solche von der Bindung mitumfaßt sein sollte.

Dabei besteht ein der Erfindung zugrundeliegendes Prinzip darin, daß die in Form einer Hybridisierungssonde eingesetzte Sensorsonde eine erhöhte Selektivität bzw. Affinität in bezug auf den Mutationsbereich des Mutationsgens bzw. des Mutationsallels bzw. des diesbezüglichen Einzelstranges gegenüber dem korrespondierenden DNA-Einzelstrang des Wildtypgens bzw. Wildtypallels aufweist, d. h. gegenüber dem korrespondierenden Genabschnitt bzw. Nukleotidbereich, welcher die zu untersuchende Mutation nicht aufweist. Die Selektivität bzw. Spezifität in bezug auf den Mutationsbereich kann in dem Fachmann an sich bekannter Weise durch eine spezielle Auswahl der Nukleotidsequenz der Hybridisierungssonde gewährleistet werden. Diesbezüglich sollte die Nukleotidsequenz bzw. Basenabfolge derart ausgewählt werden, daß die entsprechende Nukleotidsequenz zumindest im wesentlichen komplementär zu den entsprechenden Nukleotiden des Mutationsbereichs mitsamt der Mutation ist. Auf diese Weise wird die erhöhte Sensitivität bzw. Affinität bzw. Bindungsstärke der Sensorsonde in bezug auf den Mutationsbereich gewährleistet insofern, als die Anzahl der Basenpaarungen zwischen Sensorsonde einerseits und Mutationsbereich größer ist als in bezug auf eine insofern unspezifische Interaktion der Sensorsonde mit dem korrespondierenden Genabschnitt des Wildtypgens ohne Mutation. Ohne sich auf diese Theorie festlegen zu wollen, führt die höhere Bindungsaffinität insbesondere auf Basis der höheren Anzahl an bei Interaktion der Sensorsonde mit dem entsprechenden Genabschnitt bzw. DNA-Einzelstrang zu einer "festeren" Bindung bzw. Interaktion, was im Vergleich zu der Interaktion der Sensorsonde mit dem korrespondierenden Bereich des Wildtypgens zu einem höheren Schmelzpunkt, d. h. insbesondere zu einer Ablösung bzw. Dehybridisierung der Sensorsonde von dem jeweiligen DNA-Einzelstrang erst bei höheren Temperaturen führt.

Im Rahmen der vorliegenden Erfindung ist es bevorzugt, wenn die Sensorsonde eine vorzugsweise mit einer detektierbaren Substanz (Markierung), vorzugsweise mit einer bei Interaktion bzw. Bindung der Sensorsonde mit dem DNA-Einzelstrang des Mutationsgens detektierbaren Substanz, insbesondere einem Farbstoff, vorzugsweise einem Fluoreszenzfarbstoff, ausgerüstetes bzw. markiertes Nukleotidmolekül, insbesondere ein markiertes Oligo- oder Polynukleotid, ist. In diesem Zusammenhang sollte die Sensorsonde eine Größe von 3 bis 30 bp, insbesondere 5 bis 25 bp, vorzugsweise 10 bis 20 bp, aufweisen. Die zuvor angegebene Größe bezieht sich auf den Nukleotidabschnitt der Sensorsonde, welcher, wie zuvor angeführt, zumindest im wesentlichen komplementär zu dem Mutationsbereich des DNA-Einzelstranges bzw. Sondenstranges des Mutationsgens bzw. Mutationsallels sein sollte.

Die Sensorsonde sollte somit im Rahmen der vorliegenden Erfindung ausgewählt werden derart, daß die Sensorsonde eine höhere Spezifität bzw. Bindungsaffinität bzw. Selektiviät zu dem DNA-Einzelstrang des Mutationsgens bzw. Mutationsallels (Sondenstrang), insbesondere im Bereich der Genveränderung, vorzugsweise Mutation, aufweist, als zu dem hierzu korrespondierenden DNA-Einzelstrang des Wildtypgens bzw. Wildtypallels bzw. dem entsprechenden DNA-Einzelstrang in Form des wildtypischen DNA-Stranges und somit dem korrespondierenden DNA-Wildtypstrang ohne Genveränderung bzw. Mutation.

Was die Sensorsonde weiterhin anbelangt, so kann diese gemäß einer bevorzugten Ausführungsform ausgewählt werden derart, daß die Sensorsonde vorzugsweise spezifisch bzw. bevorzugt bzw. mit erhöhter Selektivität bzw. selektiv an den DNA-Einzelstrang des Mutationsgens (Sondenstrang) des EGF-Rezeptors im Bereich der Genveränderung bzw. Mutation bindet. In diesem Zusammenhang sollte die Sensorsonde zumindest im wesentlichen komplementär zum Bereich der Genveränderung des Sondenstranges ausgebildet werden.

In bezug auf die Analyse bzw. Detektion der Punktmutation T790M kann die Sensorsonde die Nukleotid- bzw. Basensequenz GGCATGAGCTGCAT-GATGAG aufweisen. Im Falle der Detektion der Punktmutation L858R kann die Sensorsonde die Nukleotidsequenz GTTTGGCCCGCCCAA aufweisen. Die vorgenannten Beispiele sind nichtbeschränkend. Vielmehr ist der Fachmann jederzeit in der Lage, spezifische Sensorsonden hinsichtlich ihrer Nukleotidsequenz bzw. ihres Nukleotidabschnitts derart auszuwählen, daß eine erhöhte Spezifität zu dem entsprechenden Genabschnitt mit der zu analysierenden bzw. zu detektierenden Mutation im Sinne der vorliegenden Erfindung gewährleistet ist.

Was die im Rahmen der vorliegenden Erfindung eingesetzte Sensorsonde weiterhin anbelangt, so kann diese beispielsweise ausgewählt werden derart, daß die Sensorsonde im Falle der insbesondere spezifischen Wechselwirkung bzw. Interaktion bzw. Bindung mit bzw. an dem DNA-Einzelstrang des Mutationsgens bzw. Mutationsallels (Sondenstrang) und/oder im Fall der insbesondere unspezifischen Bindung bzw. Interaktion mit bzw. an dem korrespondierenden DNA-Einzelstrang des Wildtypgens ein detektierbares und/oder meßbares Signal, insbesondere Fluoreszenzsignal, abzugeben imstande ist. Zudem kann die Sensorsonde ausgewählt werden derart, daß die Sensorsonde im Falle einer insbesondere wärmeinduzierten Ablösung von dem DNA-Einzelstrang des Mutationsgens bzw. Mutationsallels (Sondenstrang) oder von dem korrespondierenden DNA-Einzelstrang des Wildtypgens kein oder aber zumindest ein vermindertes oder aber ein von dem angebundenen Zustand verschiedenes detektierbares bzw. meßbares Signal abzugeben imstande ist.

Mit anderen Worten kann die Sensorsonde gemäß dieser Ausführungsform ausgebildet werden derart, daß diese zumindest im wesentlichen nur bei Interaktion bzw. Bindung bzw. Hybridisierung an dem jeweiligen DNA-Einzelstrang unter Anregung, beispielsweise unter Anregung mit elektromagnetischer Strahlung mit einer speziellen Wellenlänge bzw. mit einem speziellen Wellenlängenbereich, ein meßbares Signal, insbesondere Fluoreszenzsignal, abgibt bzw. aussendet. Entsprechend sollte die nichtgebundene Sensorsonde auch unter Anregung kein oder ein vermindertes Signal als solches oder aber, wie zuvor angeführt ein von dem gebundenen Zustand der Sensorsonde verschiedenes bzw. unterscheidbares Signal abgeben können. Insbesondere in bezug auf die nachfolgend angeführte Ausführungsform kann die Sensorsonde grundsätzlich auch derart sein, daß diese nach Anregung mit elektromagnetischer Strahlung mit einer bestimmten Wellenlänge unabhängig von der Bindung an die wt-DNA bzw. an die mt-DNA ein Signal als solches emittiert, da insbesondere gemäß der nachfolgenden Ausführungsform eine Signaldiskriminierung auf Basis eines Energietransfers auf eine andere Sonde, welche dann ein unterscheidbares Signal liefert, erfolgen kann.

Insbesondere können im Rahmen der vorliegenden Erfindung gemäß einer besonders bevorzugten Ausführungsform auch sogenannte FRET-Sonden (*Fluorescence Resonance Energy Transfer-*Sonden) eingesetzt werden. Diesbezüglich können beispielsweise unterschiedliche Farbstoffe, insbesondere Fluoreszenzfarbstoffe bzw. Fluorochrome, zum Einsatz kommen, welche an der Sensorsonde oder aber an der Sensorsonde einerseits und einem weiteren Molekül bzw. Sonde, wie nachfolgend geschildert, andererseits, gebunden sind. Eine Signalgebung kann in diesem Fall in Abhängigkeit von der Beabstandung der entsprechenden Fluoreszenzfarbstoffe zueinander und in Abhängigkeit von der Bindung an den jeweiligen DNA-Einzelstrang ausgebildet werden. Die diesbezüglichen Farbstoffe bzw. Farbstoffsysteme sind dem Fachmann als solchen wohlbekannt. Diesbezüglich erfolgt - ohne sich auf diese Theorie beschränken zu wollen - die Anregung sozusagen nicht direkt, sondern durch Anregung der einen Sonde (z. B. einer Ankersonde), insbesondere des Farbstoffes der einen Sonde (= Akzeptor), durch die andere Sonde (z. B. die Sensorsonde), insbesondere durch das Fluorophor der anderen Sonde (= Donor).

Die Anregung erfolgt somit insbesondere durch Übertragung von Energie des angeregten Fluorophors der einen Sonde auf den Farbstoff der anderen Sonde, welcher dann Energie, insbesondere in Form von sichtbarem Licht, emittiert.

Gemäß einer erfindungsgemäß bevorzugten Ausführungsform kann erfindungsgemäß zusätzlich zu der Sensorsonde mindestens eine hiervon verschiedene zweite Hybridisierungssonde (Ankersonde) eingesetzt werden.

Diesbezüglich ist es im Rahmen der vorliegenden Erfindung bevorzugt, wenn die Ankersonde ein vorzugsweise mit mindestens einer insbesondere zu der Sensorsonde komplementären bzw. kompatiblen detektierbaren Substanz (Markierung), insbesondere einem Farbstoff, vorzugsweise einem Fluoreszenzfarbstoff, ausgerüstetes bzw. markiertes Nukleotidmolekül ist. Die Ankersonde sollte ein Oligo- bzw. Polynukleotid sein bzw. ein solches Molekül bzw. einen solchen Abschnitt aufweisen. Der Nukleotidabschnitt der Ankersonde sollte eine Größe von 3 bis 50 bp, insbesondere 5 bis 45 bp, vorzugsweise 10 bis 40 bp, aufweisen. Die Nukleotidsequenz der Ankersonde sollte dabei ausgewählt werden derart, daß die Ankersonde auf demselben DNA-Einzelstrang wie die Sensorsonde zu binden imstande ist, wobei die Bindung der Ankersonde in einem benachbarten Bereich zu der zu analysierenden Genveränderung, insbesondere Mutation, und somit nicht im unmittelbaren Mutationsbereich bzw. der hierzu korrespondierenden Stelle des wildtypischen DNA-Einzelstrangs erfolgen sollte. Erfindungsgemäß sollte die Bindung der Ankersonde an dem jeweiligen DNA-Einzelstrang, insbesondere an dem Sondenstrang, erfolgen derart, daß die zu detektierenden Substanzen, insbesondere Farbstoffe, der Ankersonde einerseits und der Sensorsonde andererseits imstande sind, miteinander eine Wechselwirkung zur Bildung eines detektierbaren Signals, beispielsweise in Form von emittiertem Licht, auszubilden, insbesondere nach Art eines FRET-Paares.

In diesem Zusammenhang kann es erfindungsgemäß vorgesehen sein, daß die Ankersonde auf demselben DNA-Einzelstrang wie die Sensorsonde zu binden imstande ist, insbesondere wobei die Ankersonde in einem Abstand von 1 bis 5 bp zu der Sensorsonde zu binden imstande sein sollte, um auf diese Weise eine Wechselwirkung der detektierbaren Substanzen, insbesondere Fluoreszenzfarbstoffe, der Sensorsonde einerseits und der Ankersonde andererseits bei erfolgter Bindung bzw. Wechselwirkung beider Sonden an den DNA-Einzelstrang zu ermöglichen. Der zuvor angeführte Abstand von 1 bis 5 bp entspricht einer räumlichen Beabstandung von etwa 1 bis 10 nm. Wie zuvor angeführt, kann im Rahmen der vorliegenden Erfindung die Ankersonde ausgebildet sein derart, daß diese sowohl an dem DNA-Einzelstrang mit der Mutation (Sondenstrang) als auch an dem korrespondierenden wildtypischen DNA-Einzelstrang zu binden imstande ist. Dies ergibt sich auch dadurch, daß die jeweiligen Bindungsbereiche in bezug sowohl auf den Sondenstrang als auch auf den wildtypischen DNA-Einzelstrang im Bereich der Bindungsstelle der Ankersonde eine zumindest im wesentlichen identische Nukleotidsequenz bzw. Basenabfolge aufweisen. Vor diesem Hintergrund kann die Ankersonde an den Sondenstrang einerseits und den wildtypischen DNA-Einzelstrang andererseits mit vergleichbarer Spezifität binden, während die Sensorsonde an den Sondenstrang im Vergleich zu dem wildtypischen DNA-Einzelstrang mit erhöhter Affinität bzw. Bindungsstärke bindet. Im Rahmen der vorliegenden Erfindung ist es vorteilhaft, wenn die Sensorsonde einerseits und die Ankersonde andererseits, insbesondere die detektierbare Substanz bzw. Markierung der Sensorsonde einerseits und die detektierbare Substanz bzw. Markierung der Ankersonde andererseits, bei Bindung der Sonden ein FRET-Paar auszubilden imstande sind.

In diesem Zusammenhang besteht eine Möglichkeit der Nutzung des FRET zur Quantifizierung der zugrundeliegenden Genveränderung bzw. Mutation in der Verwendung von sogenannten LightCycler^{®}-Sonden, welche spezielle Hydrolysesonden darstellen, wobei verschiedene, jeweils mit einem Donor bzw. Akzeptor markierte Oligonukleotide (Sensorsonde und Ankersonde), die nebeneinander an der Zielsequenz (Mutationsbereich in bezug auf die Sensorsonde einerseits und diesbezüglich benachbarter Bereich in bezug auf die Ankersonde) des zu untersuchenden Genabschnitts bzw. der entsprechenden DNA-Einzelstränge binden und damit den Donor und den Akzeptor in eine für den FRET ausreichende Nähe bringen. Derartige Sondenpaare können somit zur Quantifizierung der zugrundeliegenden Mutation im Rahmen des erfindungsgemäßen Verfahrens und somit sozusagen zur Quantifizierung von PCR-Produkten eingesetzt werden. Dabei kann es im Rahmen der vorliegenden Erfindung vorgesehen sein, daß beispielsweise die Sensorsonde mit einem detektierbaren Stoff, insbesondere Fluoreszenzfarbstoff, in Form eines Donors und die Ankersonde entsprechend mit einem detektierbaren Stoff, insbesondere Fluoreszenzfarbstoff, in Form eines Akzeptors ausgerüstet bzw. markiert wird. Auch eine umgekehrte Ausrüstung bzw. Markierung, d. h. Ausrüstung der Sensorsonde mit einem Akzeptor und der Ankersonde mit einem Donor, ist im Rahmen des erfindungsgemäßen Verfahrens möglich.

Bei Bindung der Sensorsonde einerseits und der Ankersonde andererseits an den jeweiligen DNA-Strang werden - ohne sich auf diese Theorie beschränken zu wollen - Donor und Akzeptor somit in räumliche Nähe zueinander gebracht, um bei entsprechender Anregung ein FRET hervorzurufen, was zu einem detektierbaren Signal führt. Bei Ablösung der Sensorsonde bzw. der Ankersonde mit einhergehender räumlicher Beabstandung tritt auch bei Anregung kein FRET auf, so daß in diesem Fall auch kein FRET-Signal detektierbar ist. Das zugrundeliegende Prinzip des FRET bzw. der Signalausbildung mit dem spezifischen Einsatz von Donor- und Akzeptormolekülen kann somit als Maß für die Bindung von Sensor- bzw. Ankersonde an einen jeweiligen DNA-Abschnitt herangezogen werden. Dabei steigt die Fluoreszenz proportional mit der Konzentration komplementärer DNA, d. h. je mehr Sensorsonden bzw. Ankersonden in den PCR-Ansatz gebunden sind.

Eine Messung des zu detektierenden Signals, welches bei Interaktion mit dem entsprechenden DNA-Einzelstrang auftritt, kann beispielsweise am Ende einer *Annealing*-Phase eines PCR-Zyklus stattfinden. Zudem kann die Fluoreszenz, wie nachfolgend geschildert, im Rahmen einer Schmelzkurvenanalyse ermittelt bzw. erfaßt werden.

Im Falle der Detektion bzw. Analyse der Mutation T790M kann die Ankersonde insbesondere die Nukleotid- bzw. Basensequenz CACGGTGGAGGT-GAGGCAGATGC aufweisen bzw. hieraus bestehen. Im Falle der Analyse bzw. Detektion der Mutation L858R umfaßt die Nukleotid- bzw. Basensequenz der Ankersonde die Nukleotid- bzw. Basensequenz GCATGG-TATTCTTTCTCTTCCGCACCCAGC oder besteht hieraus. Der Fachmann ist jederzeit in der Lage, die für die Detektion der jeweiligen Mutation speziell auszubildende bzw. einzusetzende Ankersonde entsprechend auszuwählen bzw. maßzuschneidern.

Eine weitere Möglichkeit der Analyse bzw. der Detektion der zugrundeliegenden Genveränderung bzw. Mutation unter Ausnutzung des FRET bietet der Einsatz der erfindungsgemäß verwendeten Sensorsonde in Form eines *Molecular Beacon. Molecular Beacons* sind Oligonukleotide, die sowohl mit einem Donor als auch einem Akzeptor gekoppelt bzw. markiert sind. Die Nukleotide sind am 5'-Ende der Sonde zu den Nukleotiden am 3'-Ende komplementär, so daß sich eine für *Molecular Beacons* charakteristische, schleifenartige Sekundärstruktur ausbilden kann. Dabei kann das *Molecular Beacon* ausgebildet sein derart, daß in dem als *Stem Loop* (Stamm-Schleife) bezeichneten Zustand keine Fluoreszenz vorliegt. Durch Anlagerung der Schleifenregion an eine komplementäre DNA-Sequenz kann durch die räumliche Verlagerung von Donor und Akzeptor eine Veränderung der Fluoreszenz beobachtet bzw. detektiert werden. Im allgemeinen basieren *Molecular Beacons* auf dem sogenannten *Quencher*-Prinzip. Die diesbezüglichen Grundlagen sind dem Fachmann wohlbekannt, und der Fachmann ist jederzeit in der Lage, geeignete *Molecular Beacons* zur Anwendung im Rahmen der vorliegenden Erfindung auszuwählen.

Die Anmelderin hat weiterhin in völlig überraschender Weise gefunden, daß die Nachweisgrenze bzw. Sensitivität des erfindungsgemäßen Verfahrens zur Detektion bzw. Analyse bestimmter Mutationen dadurch weiter signifikant erhöht werden kann, daß die asymmetrische PCR sowie die diesbezügliche Verwendung mutationsspezifischer Hybridisierungssonden in spezieller Kombination mit dem Einsatz mindestens eines Blockierungsmittels durchgeführt wird. In völlig überraschender Weise kann hierdurch eine weitere Erhöhung der Sensitivität erreicht werden, wobei die Gesamtheit der erfindungsgemäß vorgesehenen Maßnahmen - Einsatz mutationsspezifischer Sensorsonden, asymmetrische PCR sowie Verwendung spezieller Blockierungsmittel - über die Wirkung der Einzelmaßnahmen hinausgeht, so daß die erfindungsgemäß realisierten Maßnahmen in völlig überraschender Weise synergistisch im Hinblick auf die Verbesserung der Sensitivität des erfindungsgemäßen Verfahrens wirken.

Im Rahmen der vorliegenden Erfindung ist es bevorzugt, wenn das Blockierungsmittel ausgewählt wird derart, daß das Blockierungsmittel eine höhere Spezifität bzw. Bindungsaffinität bzw. Selektivität in bezug auf den DNA-Einzelstrang des Wildtypgens bzw. Wildtypallels, insbesondere in bezug auf den Bereich des DNA-Einzelstrangs des Wildtypgens bzw. Wildtypallels, welcher zu dem die Genveränderung aufweisenden Genabschnitt des Mutationsgens bzw. Mutationsallels korrespondiert (wildtypischer DNA-Strang), gegenüber dem entsprechenden Mutationsgen bzw. dem diesbezüglichen DNA-Einzelstrang mit der Genveränderung bzw. Mutation (mutierter DNA-Strang) aufweist. Mit anderen Worten sollte das Blockierungsmittel derart ausgewählt werden, daß es mit höherer Spezifität bzw. selektiv an den wildtypischen DNA-Strang und insbesondere an der zu dem mutierten DNA-Einzelstrang korrespondierenden Position bzw. Stelle bindet, um auf diese Weise das unspezifische Anbinden der Sensorsonde an den wildtypischen DNA-Strang zu verringern bzw. zu verhindern. Blockierungsmittel einerseits und Sensorsonde andererseits verhalten sich somit hinsichtlich der Bindungsstellen kompetitiv, wobei die Bindung der Sensorsonde an den wildtypischen DNA-Strang verringert bzw. unterbunden wird. Auf diese Weise kann das zu detektierende Signal der Sensorsonde in bezug auf die zu erfassende Mutation weiter diskriminiert bzw. verstärkt werden, da weniger Sensorsonden unspezifisch an den wildtypischen DNA-Strang binden, sofern das Blockierungsmittel in dem PCR-Ansatz vorhanden ist. In bezug auf den mutierten DNA-Strang weist die Sensorsonde eine höhere Affinität auf als das Blockierungsmittel, so daß die Bindung der Sensorsonde an den mutierten DNA-Strang zumindest im wesentlichen nicht von dem Blockierungsmittel beeinflußt bzw. unterbunden wird.

In diesem Zusammenhang sollte die Bindung und/oder der Komplex des Blockierungsmittels mit dem DNA-Einzelstrang des Wildtypgens eine höhere Stabilität, insbesondere einen höheren Schmelzpunkt, aufweisen als die Bindung bzw. der Komplex der Sensorsonde mit dem Einzelstrang des Mutationsgens. Dies kann im Rahmen der vorliegenden Erfindung dadurch gewährleistet werden, daß die Nukleotidsequenz des Blockierungsmittels zumindest im wesentlichen komplementär zu dem den Mutationsbereich des mutierten DNA-Einzelstrangs entsprechenden nichtmutierten Bereich des wildtypischen DNA-Einzelstrangs ist.

In diesem Zusammenhang wird das Blockierungsmittel gemäß einer erfindungsgemäßen bevorzugten Ausführungsform ausgewählt derart, daß es ein Nukleotidmolekül, insbesondere ein Oligo- oder Polynukleotid, aufweist oder hieraus besteht. Dabei sollte das Blockierungsmittel eine Größe von 3 bis 30 bp, insbesondere 5 bis 25 bp, vorzugsweise 10 bis 20 bp, aufweisen.

Wie zuvor angeführt, sollte das Blockierungsmittel erfindungsgemäß ausgewählt werden derart, daß das Blockierungsmittel zumindest im wesentlichen komplementär zu dem DNA-Einzelstrang des Wildtypgens bzw. Wildtypallels, insbesondere zu dem Bereich des DNA-Einzelstrangs des Wildtypgens bzw. Wildtypallels, ist, welcher mit dem die Genveränderung aufweisenden Genabschnitt des Mutationsgens bzw. Mutationsallels korrespondiert. Der Bereich des wildtypischen DNA-Einzelstranges, welcher zu dem die Genveränderung aufweisenden Genabschnitt des Mutationsgens bzw. zu dem die Mutation aufweisenden Abschnitt des DNA-Einzelstranges korrespondiert, stellt somit gewissermaßen den zu dem Mutationsbereich analogen Abschnitt des wildtypischen DNA-Stranges des Wildtypgens dar, an welchem das Blockierungsmittel bzw. der Blocker spezifisch zu binden imstande ist.

Durch den Einsatz des spezifischen Blockierungsmittels wird somit im Ergebnis die unspezifische Bindung der Sensorsonde an den Wildtyp-Strang inhibiert bzw. vermindert, so daß nur bzw. vorrangig der mutierte DNA-Strang im Rahmen der PCR vervielfältigt wird und die eingesetzten Sonden konkurrenzlos an die mutierte DNA binden können. Das Blockierungsmittel verhindert bzw. vermindert somit einerseits die Amplifizierung des wildtypischen DNA-Stranges sowie andererseits die unspezifische Bindung der Sensorsonde an den wildtypischen DNA-Strang insbesondere im Sine einer kompetitiven bzw. konkurrierenden Bindung, was das Meßergebnis weiter verbessert. Auf diese Weise kann somit - ohne sich auf diese Theorie beschränken zu wollen - sozusagen eine Unterdrückung des wt-Signals erfolgen.

Das Blockierungsmittel sollte somit zu diesem Zweck im allgemeinen so beschaffen sein, daß es eine sehr hohe Bindungsstärke gegenüber dem Wildtyp-Einzelstrang aufweist und erst bei sehr hohen Temperaturen abschmilzt. In diesem Zusammenhang sollte das Blockierungsmittel ausgewählt werden derart, daß das Blockierungsmittel verbrückte Nukleinsäuren aufweist, in welchen der Zuckeranteil, insbesondere der Riboseanteil, chemisch modifiziert ist, insbesondere wobei der Riboseanteil eine Sauerstoff/Methylen-Brücke, vorzugsweise am C₂- und C₄-Atom des Riboseanteils, aufweist.

In diesem Zusammenhang kann das Blockierungsmittel ausgewählt werden derart, daß das Blockierungsmittel ein Nukleinsäure-Analogon in Form einer blockierten Nukleinsäure (*Locked Nucleic Acid* bzw. LNA) ist. Bei derartig blockierten Nukleinsäuren handelt es sich um insbesondere strukturell weniger flexibel und somit um drehsteife Moleküle, welche an den DNA-Strang unter Ausbildung eines erhöhten Schmelzpunktes spezifisch binden bzw. hybridisieren.

In diesem Zusammenhang sollte in bezug auf die Detektion bzw. Analyse der Mutation T790M das Blockierungsmittel, insbesondere in Form eines LNA, die Nukleotidsequenz TGAGCTGCGTGATG aufweisen oder hieraus bestehen; hinsichtlich der Detektion bzw. Analyse der Mutation L858R sollte das Blockierungsmittel, insbesondere in Form eines LNA, die Nukleotidsequenz GCCAGCCCAAAATCT aufweisen oder aus dieser Nukleotidsequenz bestehen.

Im Rahmen der vorliegenden Erfindung ist es gleichermaßen möglich, daß das Blockierungsmittel ein weiteres Nukleinsäure-Analogon ist, insbesondere wobei das Blockierungsmittel ein peptidisches und/oder peptidbasiertes Rückgrat aufweist und/oder insbesondere wobei das Blockierungsmittel eine Peptidnukleinsäure (*Peptide Nucleic Acid* bzw. *PNA)* ist. Hierbei handelt es sich um ein DNA-Analogon, bei welchem das Zucker-Phosphat-Rückgrat durch ein Pseudopeptid ersetzt ist. Auch derartige Peptid-Nukleinsäuren führen zu einer höheren Affinität der Bindung zur komplementären DNA-Sequenz, was zur Ausbildung festerer bzw. stabilerer Bindungen führt, einhergehend mit einer Schmelzpunkterhöhung.

Das erfindungsgemäße Verfahren kann gemäß einer bevorzugten Ausführungsform derart durchgeführt werden, daß zum Nachweis bzw. zur Detektion der Genveränderung, insbesondere der Mutation, insbesondere im Anschluß an die Polymerase-Kettenreaktion (*Polymerase Chain Reaction* bzw. PCR) eine Schmelzkurve aufgenommen bzw. eine Schmelzkurvenanalyse durchgeführt wird. In diesem Zusammenhang können die Reaktionsansätze langsam erhitzt werden, beispielsweise bis auf 95 °C. An dem Punkt bzw. bei der Temperatur, an dem sich 50 % der Sensorsonden von den entstandenen PCR-Produkten gelöst haben, fällt die Fluoreszenz deutlich ab. Sofern die Sensorsonde spezifisch an den mutierten DNA-Einzelstrang gebunden hat, erfolgt die Dehybridisierung der Sensorsonde bei einer höheren Temperatur im Vergleich zu einer Bindung an der entsprechenden Wildtyp-Sequenz bzw. dem entsprechenden Wildtyp-DNA-Einzelstrang. Die entsprechende Analyse der Schmelzkurven läßt somit Rückschlüsse auf das Vorhandensein einer spezifischen Mutation zu.

Insbesondere liegt die Abschmelztemperatur der Sensorsonde insbesondere am Ende eines PCR-Laufes niedriger bei Bindung an die wt-DNA als bei Bindung an mt-DNA, für welche die Sensorsonde spezifisch ist.

Diesbezüglich kann die Abspaltung bzw. Dehybridisierung insbesondere der Sensorsonde von dem jeweiligen DNA-Einzelstrang des Mutationsgens bzw. des Wildtypgens erfaßt werden, insbesondere wobei die Erfassung photometrisch, insbesondere durch Messung der Fluoreszenz, erfolgt. Wie zuvor dargestellt, führt die Hybridisierung bzw. die Abspaltung der Sensorsonde von dem jeweiligen DNA-Einzelstrang zu einer Abnahme der Fluoreszenz, wobei der diesbezügliche Schmelzpunkt aufgrund der geringeren Bindungsstärke bzw. Affinität in bezug auf den Wildtyp-DNA-Einzelstrang geringer bzw. niedriger ausfällt als in bezug auf den DNA-Einzelstrang mit der entsprechenden Mutation. Durch den Einsatz der mutationsspezifischen Sensorsonden kann demnach festgestellt werden, ob in einer Probe mutierte Genformen vorliegen und um welche Mutationen es sich im konkreten Fall handelt. Zur besseren Analyse der Schmelzkurven können diese in Form der ersten mathematischen Ableitung dargestellt werden; insbesondere stellen die Maxima der betreffenden ersten mathematischen Ableitung die jeweiligen Schmelzpunkte dar.

Im Rahmen des erfindungsgemäßen Verfahrens kann somit derart vorgegangen werden, daß anhand des oder der Schmelzpunkte und/oder Schmelzpunktbereiche der Schmelzkurve auf das Vorliegen einer Genveränderung, insbesondere Mutation, geschlossen wird und im Falle des Vorliegens einer Mutation die Art der Mutation bestimmt wird. Mit anderen Worten kann auf Basis der Analyse bzw. Zuordnung (d. h. Zuordnung über eine Referenz, wie nachfolgend noch beschrieben) der Schmelzpunkte bzw. Schmelzbereiche festgestellt werden, ob und bejahendenfalls welche konkrete Mutation in der Probe vorliegt.

In diesem Zusammenhang kann beispielsweise derart vorgegangen werden, daß im Rahmen der Schmelzkurvenanalyse parallele Vergleichs- bzw. Referenzansätze mitgeführt bzw. analysiert werden oder aber alternativ diese Vergleichs- bzw. Referenzansätze vorab und/oder unabhängig vermessen worden sind (d. h. mit anderen Worten das Verfahren über eine Referenz standardisiert wird). So kann z. B. mindestens ein Referenzansatz auf Basis einer wildtypischen DNA bzw. eines Wildtypgens bzw. -allels in einem insbesondere parallelen Ansatz mitgeführt bzw. vorab vermessen bzw. standardisiert werden. Der Referenzansatz kann ohne Blockierungsmittel mitgeführt bzw. vorgelegt werden, d. h. es kann ein weiterer Referenzansatz des Wildtypgens ohne Blockierungsmittel mitgeführt werden. Gleichermaßen kann es vorgesehen sein, daß mindestens ein Referenzansatz auf Basis einer zu analysierenden Genveränderung bzw. Mutation und/oder einer definierten bzw. vorbekannten Genveränderung bzw. Mutation in einem insbesondere parallelen Ansatz mitgeführt wird bzw. vorab vermessen bzw. standardisiert wird; hierbei kann es sich insbesondere um solche Referenzansätze handeln, welche Mutationsgene bzw. Mutationsallele auf Basis der T790M-Mutation und/oder auf Basis der L858R-Mutation enthalten.

Ein Vergleich der anhand der zu analysierenden Proben einerseits und der anhand der Referenzansätze bzw. -messungen erhaltenen Schmelzkurven ermöglicht dann eine Aussage über das Vorliegen einer speziellen Mutation in der Probe (d. h. Prüfung auf Vorliegen einer Genveränderung bzw. Mutation und bejahendenfalls der Art der Genveränderung bzw. Mutation). So kann im allgemeinen dann von dem Vorliegen einer Genveränderung bzw. Mutation ausgegangen werden, wenn sich in bezug auf den Referenzansatz mit dem Wildtyp unterschiedliche, insbesondere bei höheren Temperaturen auftretende Schmelzpunkte bzw. Schmelzbereiche für die zu untersuchende Probe ergeben. Das Vorliegen einer Genveränderung bzw. Mutation kann auch anhand eines Vergleichs der Schmelzkurve mit derjenigen des jeweiligen Referenzansatzes mit dem betreffenden Mutationsgen erfolgen, wobei das Vorliegen von zumindest im wesentlichen gleichen Schmelzpunkten bzw. -bereichen als Anzeichen für das Vorhandensein gleicher Genveränderungen bzw. Mutationen herangezogen werden kann.

Grundsätzlich ist es im Rahmen der vorliegenden Erfindung gemäß einer weniger bevorzugten Ausführungsform aber auch möglich, die Analyse auf das Vorliegen einer Mutation ohne Referenzansatz durchzuführen, insbesondere bei bereits bekannten Schmelzpunkten bzw. -bereichen oder aber bei vorab erfolgter Standardisierung (insbesondere wie zuvor beschrieben).

Nachfolgend wird eine Analyse bzw. Auswertung rein exemplarisch und nichtbeschränkend auf Basis einer Versuchsdurchführung mit einem parallelen Wildtyp-Referenzansatz weiter veranschaulicht:

Was die Analyse der Schmelzkurven anbelangt, so wird im allgemeinen die Schmelzkurve mit der höchsten spezifischen Schmelztemperatur bzw. dem höchsten Schmelztemperaturbereich dem Mutationsgen zugeordnet, d. h. das Vorliegen verschiedener Schmelzkurven mit unterschiedlichen Schmelzpunkten bzw. -bereichen kann als Beleg bzw. Indiz für das Vorliegen einer Mutation herangezogen werden, wobei die Kurve mit dem höheren Schmelzpunkt bzw. Schmelzbereich auf die festere Bindung der Sensorsonde an den mutierten DNA-Einzelstrang und somit auf die mutierte Form zurückzuführen ist. Zudem kann bei Vorliegen mehrerer Schmelzkurven die Schmelzkurve mit der niedrigeren spezifischen Schmelztemperatur bzw. dem niedrigeren Schmelztemperaturbereich dem Wildtypgen zugeordnet werden, wobei die diesbezügliche Schmelzkurve mit der niedrigeren Schmelztemperatur bzw. dem niedrigeren Schmelztemperaturbereich ursächlich durch die weniger stark ausgebildete und unspezifische Bindung der Sensorsonde an den nichtmutierten Abschnitt des wildtypischen DNA-Einzelstranges hervorgerufen wird. Sofern in der zu analysierende Probe auch wildtypische DNA vorhanden ist, kann sich für die Schmelzkurve der genveränderten bzw. mutierten Form gleichermaßen ein bei niedrigen Temperaturen vorliegender Schmelzpunkt bzw. - bereich ausbilden, welcher aufgrund der erfindungsgemäß durchgeführten Maßnahmen im allgemeinen jedoch ein geringeres Signalmaximum aufweist (z. B. Unterdrückung der Anbindung der Sensorsonde und/oder Unterdrückung der Amplifizierung).

Zudem ist es im Rahmen der vorliegenden Erfindung neben der zuvor angeführten Ermittlung bzw. Analyse, ob eine Mutation in einer Probe vorliegt und um welche Mutation es sich hierbei handelt, gleichermaßen möglich, eine Aussage darüber zu treffen, ob es sich bei der Mutation um eine homozygote bzw. heterozygote Genveränderung handelt. Hierbei sollte in bezug auf die Probe von einem zumindest im wesentlichen nicht mit Wildtypgenen verunreinigten Ansatz ausgegangen werden, welcher das zu analysierende Mutationsgen enthält. So kann es im Rahmen des erfindungsgemäßen Verfahrens vorgesehen sein, daß die Schmelzkurve mit dem höchsten Schmelzpunkt und/oder dem höchsten Schmelzbereich einer homozygoten Genveränderung zugeordnet wird für den Fall, daß die Schmelzkurve einen einzigen Schmelzpunkt bzw. Schmelzbereich aufweist. Zudem kann im Rahmen des erfindungsgemäßen Verfahrens derart vorgegangen werden, daß die Schmelzkurve mit dem höchsten Schmelzpunkt bzw. dem höchsten Schmelzbereich einer heterozygoten Genveränderung zugeordnet wird für den Fall, daß die Schmelzkurve zwei voneinander verschiedene Schmelzpunkte bzw. Schmelzbereiche aufweist.

Zum Nachweis von homozygoten bzw. heterozygoten Genveränderungen in einer Probe, welche auch das Wildtypgen enthält, kann beispielsweise derart vorgegangen werden, daß ein erster PCR-Ansatz der Probe ohne Blockierungsmittel und ein zweiter paralleler PCR-Ansatz mit Blockierungsmittel, gegebenenfalls mit einem weiteren Referenzansatz auf Basis des Wildtypgens, durchgeführt und analysiert werden. Über einen Vergleich der Ausbildung der Schmelzpunkte bzw. -bereiche insbesondere hinsichtlich der entsprechenden Signalmaxima der Wildtypgene bzw. Wildtypallele in den Proben mit bzw. ohne Blockierungsmittel kann eine Analyse auf das Vorliegen einer homozygot bzw. heterozygot ausgebildeten Mutation erfolgen.

Insbesondere aufgrund der spezifischen Kombination aller erfindungsgemäßen Maßnahmen ist es im Rahmen der vorliegenden Erfindung möglich, daß eine mindestens 10fache, insbesondere mindestens 50fache, vorzugsweise mindestens 100fache, bevorzugt mindestens 500fache, besonders bevorzugt mindestens 1.000fache Verstärkung bzw. Amplifizierung der Detektion in bezug auf den die Genveränderung aufweisenden Genabschnitt, insbesondere DNA-Abschnitt, des Mutationsgens bzw. des damit einhergehenden (Fluoreszenz)Signals erfolgt. Insbesondere ist es im Rahmen der vorliegenden Erfindung möglich, daß eine mindestens 10fache, insbesondere mindestens 50fache, vorzugsweise mindestens 100fache, bevorzugt mindestens 500fache, besonders bevorzugt mindestens 1.000fache Verstärkung und/oder Amplifizierung des mit dem die Genveränderung aufweisenden Genabschnitt, insbesondere DNA-Abschnitt, des Mutationsgens im Zusammenhang stehenden Meßsignals erfolgt.

Im Ergebnis ist es im Rahmen der vorliegenden Erfindung gelungen, auf Basis der Kombination der zuvor genannten Maßnahmen einen äußerst sensitiven Mutationsnachweis bereitzustellen. Im Rahmen der vorliegenden Erfindung kann eine Mutation analysiert bzw. nachgewiesen werden, selbst wenn in der Probe nur sehr geringe Mengen an mutierter DNA vorliegen.

Das erfindungsgemäße Verfahren eignet sich gleichermaßen dazu, Verlaufskontrollen auf Basis des der Probe zugrundeliegenden Körpermaterials und insbesondere in vorzugsweise peripherem Blut durchzuführen, was eine leichte Nachverfolgung des Erkrankungsverlaufes auf molekularer Ebene ermöglicht. Auf Basis des erfindungsgemäßen Verfahrens, gegebenenfalls in Kombination mit weiteren Aufreinigungsmethoden, welche insbesondere auf eine spezifische Aufreinigung bzw. Isolation der zu untersuchenden DNA aus der Probe abstellen und welche dem Fachmann als solche wohlbekannt sind, ist es im Rahmen der vorliegenden Erfindung möglich, zirkulierende Tumorzellen in Vollblut nachzuweisen.

Das erfindungsgemäße Verfahren eignet sich somit dazu, Aussagen zur Diagnose der Tumor- bzw. Krebserkrankung bzw. zur Ermittlung des Risikos, an einer Tumor- bzw. der Krebserkrankung zu erkranken, bzw. zur Prognose eines Krankheitsverlaufs der Tumor- bzw. Krebserkrankung bzw. zur Prognose von individuellen Arzneimittelwirkungen bei Behandlung der Tumor- und/oder Krebserkrankung zu treffen. So kann beispielsweise und in nicht beschränkender Weise einem Patienten bzw. Probanden ein erhöhtes Risiko zugeordnet werden, an einem Bronchialkarzinom zu erkranken, sofern dieser eine oder mehrere der vorgenannten Mutationen aufweist, welche auf Basis des erfindungsgemäßen Verfahrens nachgewiesen werden können. Gleichermaßen kann eine Krankheitsprognose bzw. ein Krankheitsverlauf aufgenommen bzw. analysiert werden, wobei diesbezüglich dem Patienten bzw. Probanden über einen bestimmten Zeitraum Proben entnommen und auf Basis des erfindungsgemäßen Verfahrens analysiert werden können und der Nachweis bzw. die Detektion der entsprechenden Mutationen für das Vorliegen von Tumorzellen in der Probe herangezogen werden können.

Gleichermaßen ist es im Rahmen der vorliegenden Erfindung möglich, auf Basis der konkreten Analyse der zugrundeliegenden Mutation den Therapieansatz insbesondere in bezug auf die spezifische Medikation zu optimieren. So können auf Basis der vorgefundenen Mutationen die jeweils spezifischen bzw. diesbezüglich optimal wirkenden Medikamente eingesetzt werden, was zu einer weiterführenden Individualisierung und Spezifierung der Medikation bzw. Therapie, einhergehend mit einer höheren therapeutischen Wirksamkeit, führt. So kann bei vorliegen der T790M-Mutation, gegebenenfalls in Kombination mit der L858R-Mutation, die Applikation bzw. Verabreichung der zuvor beschriebenen Inhibitoren der zweiten Generation indiziert sein, während bei Vorliegen der L858R-Mutation die Gabe der zuvor beschriebenen Inhibitoren der ersten Generation indiziert sein kann.

Wie zuvor angeführt, kann das erfindungsgemäße Verfahren zur Mutationsanalyse bei Bronchialkarzinomen und insbesondere bei dem nichtkleinzelligen Lungenkarzinom bzw. NSCLC mit der diesbezüglichen Involvierung des EGF-Rezeptors eingesetzt werden.

Die vorliegende Erfindung betrifft somit - gemäß einem bevorzugten Aspekt der vorliegenden Erfindung - ein Verfahren zum Nachweis bzw. zur Detektion einer Genveränderung, insbesondere Mutation, in einem für den EGF-Rezeptor kodierenden Gen, wobei der EGF-Rezeptor insbesondere im Zusammenhang mit einer Tumor- bzw. Krebserkrankung, insbesondere einem Lungenkarzinoms, wie dem nichtkleinzelligen Lungenkarzinom (NSCLC), steht, insbesondere wobei das die Genveränderung aufweisende Gen (Mutationsgen) zusammen mit weiteren, für das Protein kodierenden, jedoch keine Genveränderung aufweisenden Genen (Wildtypgenen) vorliegt. Das Verfahren gemäß diesem Aspekt der vorliegenden Erfindung zeichnet sich gleichermaßen dadurch aus, daß es mittels asymmetrischer Polymerase-Kettenreaktion unter kombinierter Verwendung mindestens einer detektierbaren mutationspezifischen Hybridisierungssonde (Sensorsonde) einerseits und mindestens eines die Anbindung der Sensorsonde an die Wildtypgene inhibierenden wildtypspezifischen Blockierungsmittels durchgeführt wird, insbesondere so daß eine selektive Verstärkung und/oder Amplifizierung der Detektion in bezug auf einen die Genveränderung aufweisenden Genabschnitt, insbesondere DNA-Abschnitt, des Mutationsgens erfolgt.

Die erfindungsgemäßen Verfahren gemäß den obigen Aspekten können als solche auf Basis von dem Fachmann an sich bekannten Methoden unter Einbezug der diesbezüglichen Apparaturen und Meßeinrichtungen durchgeführt werden. Beispielsweise kann die Polymerase-Kettenreaktion sowie die Aufnahme und Analyse der entsprechenden Schmelzkurven unter Verwendung einer LightCycler^{®}-480-Apparatur der Firma F. Hoffmann-La Roche Ltd. durchgeführt werden.

Die vorliegende Erfindung betrifft zudem - gemäß einem **zweiten** Aspekt der vorliegenden Erfindung - eine Zusammensetzung insbesondere zur Verwendung im Rahmen einer asymmetrischen Polymerase-Kettenreaktion, vorzugsweise zum Nachweis und/oder zur Detektion mindestens einer Genveränderung, insbesondere Mutation, in einem Gen, vorzugsweise in einem für ein mit einer Tumor- und/oder Krebserkrankung in Verbindung stehenden Protein kodierenden Gen, insbesondere wobei das die Genveränderung aufweisende Gen (Mutationsgen) zusammen mit weiteren, für das Protein kodierenden, jedoch keine Genveränderung aufweisenden Genen (Wildtypgenen) vorliegt, wobei die Zusammensetzung in Kombination enthält:
(a) eine detektierbare mutationspezifische Hybridisierungssonde (Sensorsonde);
(b) einen ersten Primer, welcher zumindest im wesentlichen spezifisch mit dem DNA-Einzelstrang des Mutationsgens (Sondenstrang) bindet, mit welchem die Sensorsonde zu interagieren imstande ist;
(c) einen zweiten Primer, welcher zumindest im wesentlichen spezifisch mit dem zum Sondenstrang komplementären DNA-Einzelstrang des Mutationsgens (Komplementärstrang) zu interagieren imstande ist;
(d) ein die Anbindung der Sensorsonde an die Wildtypgene inhibierendes wildtypspezifisches Blockierungsmittel;
wobei der Gehalt an dem ersten Primer (b) in der Zusammensetzung größer ist als der Gehalt an dem zweiten Primer (c).

Was die erfindungsgemäße Zusammensetzung anbelangt, so kann diese (a) die Hybridisierungssonde bzw. Sensorsonde in einer Menge von 0,01 bis 5 pmol/µl, insbesondere 0,05 bis 3 pmol/µl, vorzugsweise 0,1 bis 1 pmol/µl, bezogen auf die Zusammensetzung, enthalten.

Zudem kann die erfindungsgemäße Zusammensetzung (b) den ersten Primer in einer Konzentration von 0,05 bis 10 pmol/µl, insbesondere 0,1 bis 5 pmol/µl, vorzugsweise 0,2 bis 2 pmol/µl, bezogen auf die Zusammensetzung, enthalten.

Weiterhin kann die erfindungsgemäße Zusammensetzung (c) den zweiten Primer in einer Konzentration von 0,005 bis 5 pmol/µl, insbesondere 0,01 bis 2 pmol/µl, vorzugsweise 0,03 bis 0,5 pmol/µl, bezogen auf die Zusammensetzung, enthalten.

Die erfindungsgemäße Zusammensetzung sollte (a) den ersten Primer und (b) den zweiten Primer in einem mengenmäßigen Verhältnis des (a) ersten Primers zu (b) den zweiten Primer ((a) : (b)), bezogen auf die Zusammensetzung, im Bereich von 1.000 : 1 bis 1,05 : 1, insbesondere 100 : 1 bis 1,5 : 1, vorzugsweise 10 : 1 bis 2 : 1, enthalten.

Die Zusammensetzung kann zudem (d) das Blockierungsmittel in einer Konzentration von 0,005 bis 4 pmol/µl, insbesondere 0,01 bis 1 pmol/µl, vorzugsweise 0,015 bis 0,1 pmol/µl, bezogen auf das Gesamtvolumen der Zusammensetzung, enthalten.

Zudem kann die erfindungsgemäße Zusammensetzung insbesondere eine (e) Ankersonde, insbesondere wie zuvor definiert, enthalten, insbesondere in einer Menge von 0,01 bis 5 pmol/µl, insbesondere 0,05 bis 3 pmol/µl, vorzugsweise 0,1 bis 1 pmol/µl, bezogen auf die Zusammensetzung.

Bei der Zusammensetzung handelt es sich insbesondere um eine wäßrige Lösung bzw. Dispersion. In diesem Zusammenhang kann die Zusammensetzung insbesondere PCR-reines Wasser enthalten. Zudem kann die Zusammensetzung sogenanntes *480-probes-Master* enthalten.

Die vorliegende Zusammensetzung kann anwendungsfertig zubereitet bzw. portioniert sein sowie zur Aufbewahrung bzw. Lagerung gekühlt bzw. eingefroren werden.

Gleichermaßen ist es im Rahmen der vorliegenden Erfindung auch möglich, daß die Zusammensetzung zumindest teilweise als räumlich voneinander getrennte Komponenten, insbesondere als *Kit-of-Parts,* vorliegt, wobei die Komponenten (a) bis (d) und gegebenenfalls (e) zumindest teilweise voneinander getrennt vorliegen können. Diesbezüglich können die Komponenten bzw. Bestandteile beispielsweise unmittelbar vor Versuchsdurchführung zum Erhalt einer anwendungsfertigen Zusammensetzung zusammengeführt werden.

Schließlich betrifft die vorliegende Erfindung - gemäß einem **dritten** Aspekt der vorliegenden Erfindung - die Verwendung der erfindungsgemäßen Zusammensetzung, wie zuvor definiert, zum Nachweis und/oder zur Detektion mindestens einer Genveränderung, insbesondere Mutation, in einem Gen, vorzugsweise in einem für ein mit einer Tumor- und/oder Krebserkrankung in Verbindung stehenden Protein kodierenden Gen, insbesondere wobei das die Genveränderung aufweisende Gen (Mutationsgen) zusammen mit weiteren, für das Protein kodierenden, jedoch keine Genveränderung aufweisenden Genen (Wildtypgenen) vorliegt, insbesondere im Rahmen einer asymmetrischen Polymerase-Kettenreaktion.

Die vorliegende Erfindung wird anhand der Figurendarstellung weiter und rein exemplarisch veranschaulicht, ohne die Erfindung jedoch hierauf zu beschränken. Es zeigen:
- Fig. 1: einen schematischen Aufbau des EGF-Rezeptors mit der entsprechenden Lokalisierung der Mutationen.
- Fig. 2: das Nachweisprinzip unter Verwendung von Hybridisierungssonden auf Basis von mutationsspezifischen Sensorsonden einerseits und Ankersonden andererseits sowie die Ausbildung eines FRET-Signals.
- Fig. 3: das Prinzip der asymmetrischen PCR.
- Fig. 4: das Prinzip der Inhibierung des Wildtyps durch das erfindungsgemäß eingesetzte Blockierungsmittel.
- Fig. 5: eine Darstellung von spezifischen Schmelzkurven unter Verwendung einer LightCycler^{®} 480-Apparatur.
- Fig. 6: eine Darstellung von spezifischen Schmelzkurven der L858R-Mutation unter zusätzlicher Verwendung eines Blockierungsmittels.
- Fig. 7: eine Darstellung von spezifischen Schmelzkurven der T790M-Mutation unter Zugabe eines Blockierungsmittels.

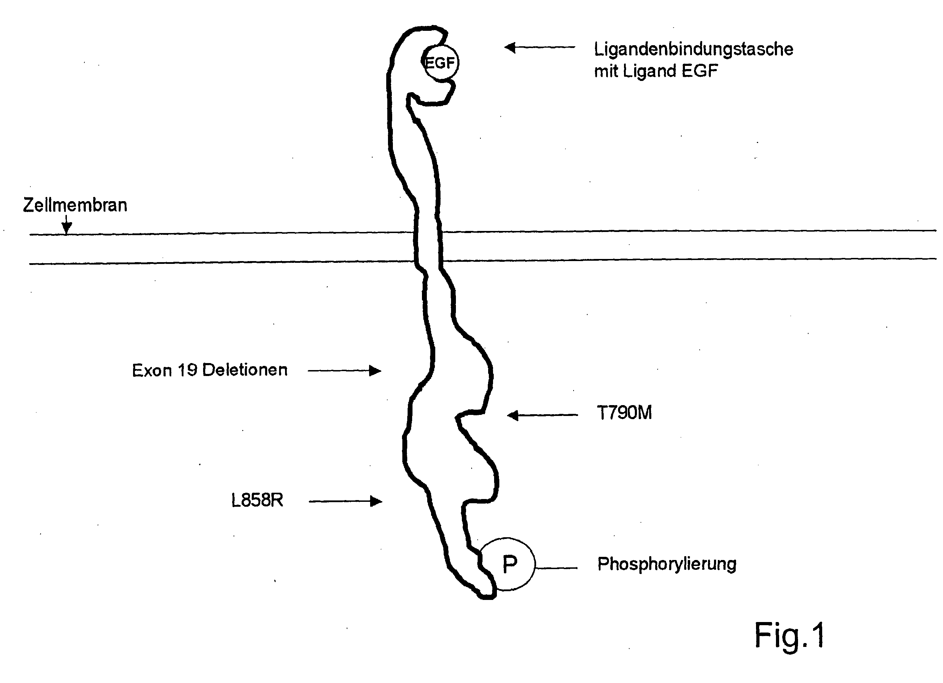

Fig. 1 zeigt den Aufbau des EGF-Rezeptors. Der Wachstumsrezeptor besteht aus einer extrazellulären und einer intrazellulären Domäne sowie einer Transmembrandomäne. Der Ligand EGF wird in der Ligandenbindungstasche des Rezeptors im extrazellulären Bereich gebunden. Im intrazellulären Bereich befindet sich die Phosphorylierungsstelle sowie die Deletionen in E-xon 19 und die Punktmutationen L858R und T790M.

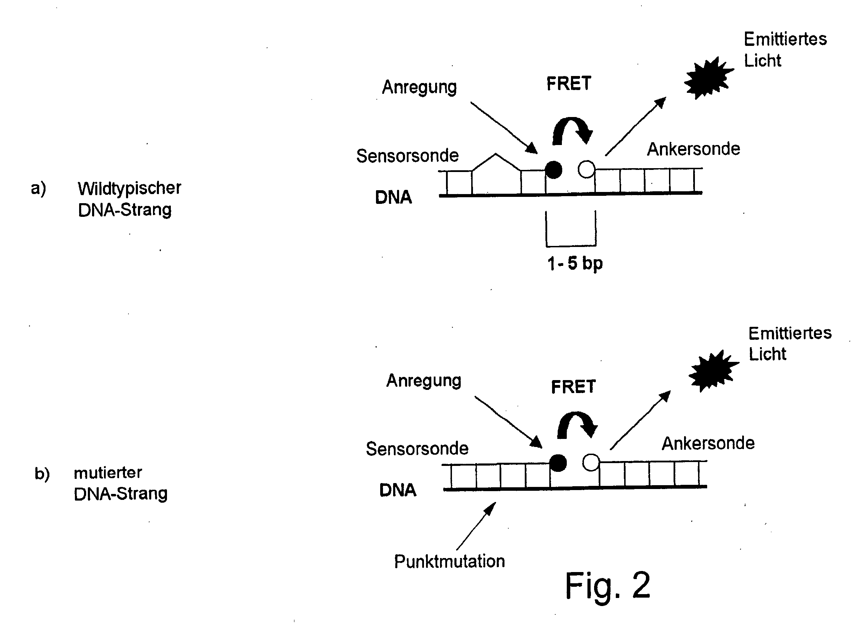

Weiterhin verdeutlicht Fig. 2 das Nachweisprinzip unter Verwendung von Hybridisierungssonden. In diesem Zusammenhang zeigt Fig. 2 a) die Bindung der beiden Hybridisierungssonden an einen DNA-Einzelstrang mit Wildtypsequenz. Da die Sensorsonde spezifisch an den mutierten Strang bindet, kann sie in diesem Fall an den Wildtyp-Strang nur unspezifisch hybridisieren. Der Fiuoreszenzfarbstoff der Sensorsonde wird durch Licht angeregt, die Energie mittels FRET (Fluoreszenz Resonanz-Energie-Transfer) auf den Fluoreszenzfarbstoff der Ankersonde übertragen, und das emittierte Licht kann gemessen werden. Im Rahmen der vorliegenden Erfindung ist es aber ebenfalls möglich, daß - wie zuvor angeführt - die Sensorsonde einerseits und die Ankersonde andererseits derart jeweils ausgewählt bzw. markiert werden, daß - gewissermaßen umgekehrt - ein Energieübertrag von der Ankersonde auf die Sensorsonde stattfinden kann. In diesem Fall liegen somit eine Anregung des Fluoreszenzfarbstoffes der Ankersonde und eine Energieübertragung mittels FRET auf die Sensorsonde vor. Damit der Energietransfer stattfinden kann, sollte der Basenabstand zwischen den Sonden nicht mehr als 5 bp betragen. Fig. 2 b) zeigt die Bindung der jeweiligen Hybridisierungssonden - nämlich Sensorsonde einerseits und Ankersonde andererseits - an einen DNA-Einzelstrang mit Mutationssequenz bzw. der nachzuweisenden Genveränderung. Hier bindet die Sensorsonde spezifisch im Mutationsbereich. Durch Anregung des Fluoreszenzfarbstoffes der Sensorsonde und Übertragung der Energie mittels FRET auf den Farbstoff der Ankersonde wird ebenfalls ein Signal emittiert, welches gemessen werden kann.

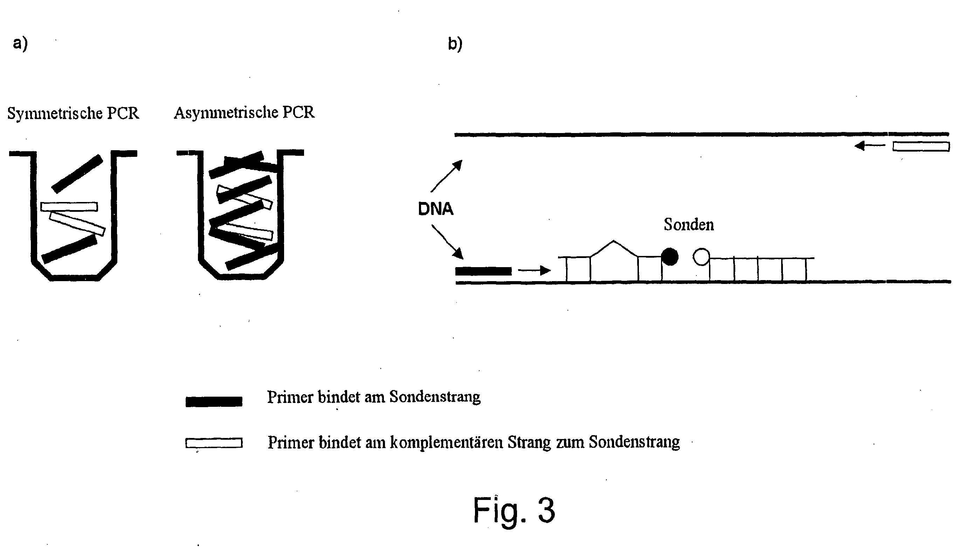

Weiterhin verdeutlicht Fig. 3 das Prinzip der erfindungsgemäß eingesetzten asymmetrischen PCR. In diesem Zusammenhang zeigt Fig. 3 a) daß - im Gegensatz zur symmetrischen PCR - das Primerverhältnis bei der asymmetrischen PCR nicht gleich ist. Die Konzentration des Primers, der am Sondenstrang bindet, wird erhöht (hier schwarz dargestellt). Fig. 3 b) zeigt eine Übersicht, wonach der schwarz markierte Primer an den Sondenstrang bindet, während der weiß dargestellte Primer an den komplementären DNA-Einzelstrang bindet.

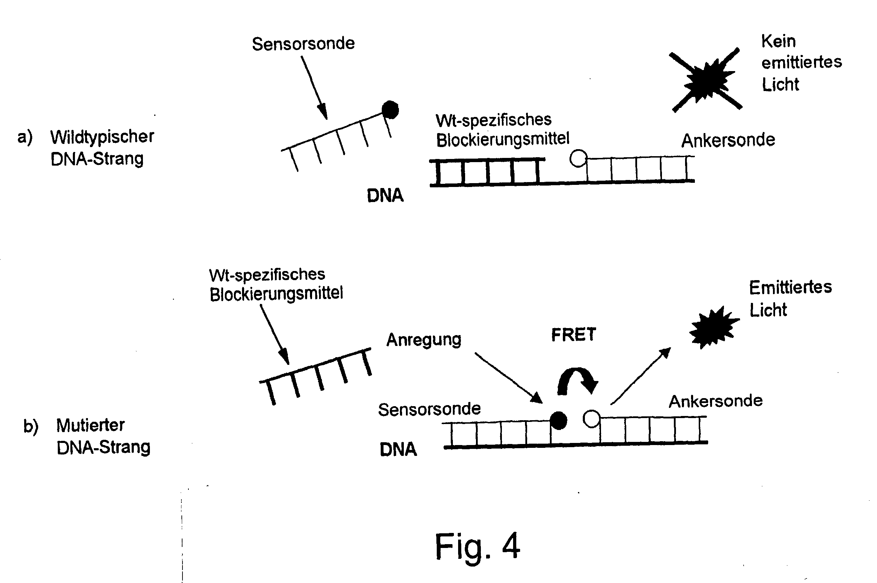

Fig. 4 veranschaulicht das Prinzip der Inhibierung des Wildtyp-DNA-Einzelstranges durch das erfindungsgemäß eingesetzte Blockierungsmittel. Fig. 4 a) zeigt die Bindung des für den Wildtyp (wt) spezifischen Blockierungsmittels an den Wildtyp-DNA-Einzelstrang. Somit kann die Sensorsonde an dieser Stelle nicht mehr hybridisieren. Die wt-DNA wird zudem nicht mehr amplifiziert und emittiert durch das fehlende FRET auch kein Signal. Fig. 4 b) zeigt die Situation, wonach an dem mutierten DNA-Einzelstrang das Blockierungsmittel nicht binden kann, jedoch die Sensorsonde. Die mutierte DNA wird amplifiziert und emittiert ein Signal, insbesondere in Form von Licht, welches gemessen werden kann.

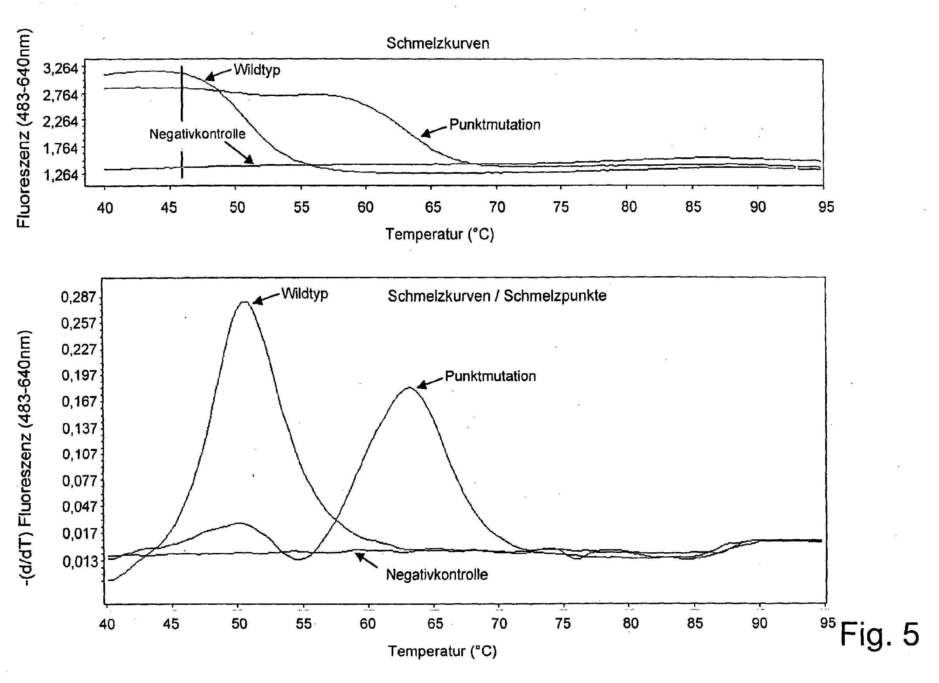

Fig. 5 zeigt eine Darstellung der spezifischen Schmelzkurven. Nach der eigentlichen PCR findet eine Schmelzkurvenanalyse statt. Im oberen Teil der Figur ist die Abnahme der Fluoreszenz mit zunehmender Schmelztemperatur bzw. Probentemperatur dargestellt. Im unteren Teil des Bildes ist der Wendepunkt der gemessenen Fluoreszenzabnahme als Kurve dargestellt. Die Kurve mit dem ausschließlich linken Maximum (bei niedrigen Temperaturen) zeigt eine wildtypische Probe, die flache Kurve eine Negativkontrolle und die Kurve mit dem rechten Maximum (bei hohen Temperaturen) zeigt eine heterozygote Probe an. Eine heterozygote Probe zeichnet sich dadurch aus, daß sie ein wildtypisches und ein mutiertes Allel besitzt. Aus diesem Grund zeigen heterozygote Proben auch einen sogenannten "Doppelpeak", das heißt zwei Schmelzpunkte bzw. zwei Schmelzpunktbereiche, auf

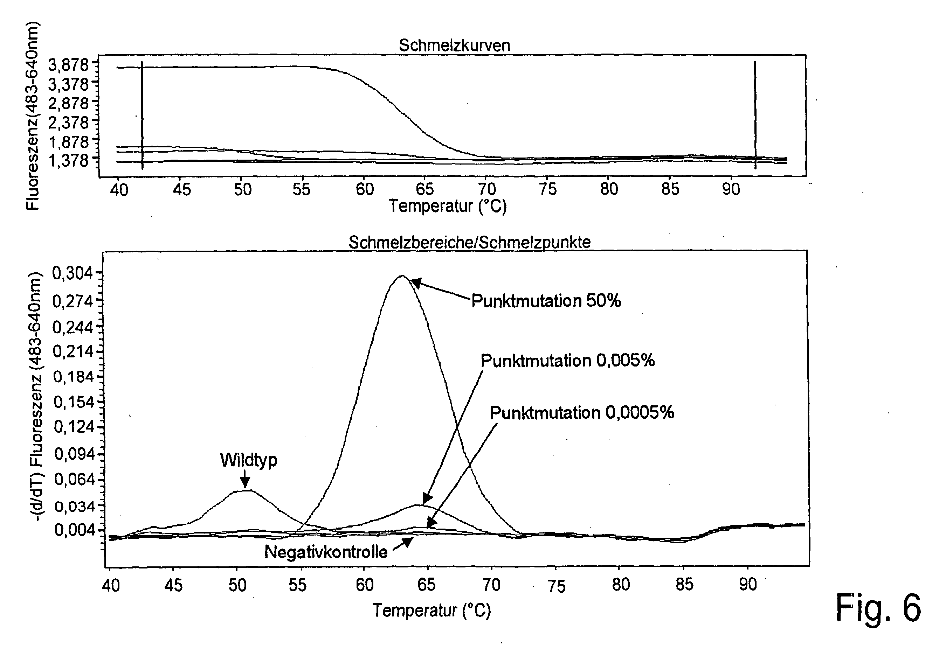

Fig. 6 zeigt die Darstellung der spezifischen Schmelzkurven für die L858R-Mutation. Während der Schmelzkurvenanalyse wird die Abnahme der Fluoreszenz gemessen. Das Signal des Wildtyps (linkes Maximum) wird in Proben, die ausschließlich Wildtyp-DNA beinhalten, nahezu komplett inhibiert. Proben mit der in Rede stehenden Punktmutation, unabhängig ob homozygot oder heterozygot, sind durch einen Peak bzw. ein Maximum mit höherer Schmelztemperatur nachzuweisen (rechte Maxima.) Proben, die einen minimalen Gehalt an mutierter DNA enthalten, weisen ebenfalls eine Kurve bei der spezifischen Schmelztemperatur der Mutanten auf. Hier läßt sich ein Anteil von bereits 0,0005 % L858R-mutierter Zellen in einem Wildtypgemisch, bezogen auf den Gesamtzellgehalt, detektieren.

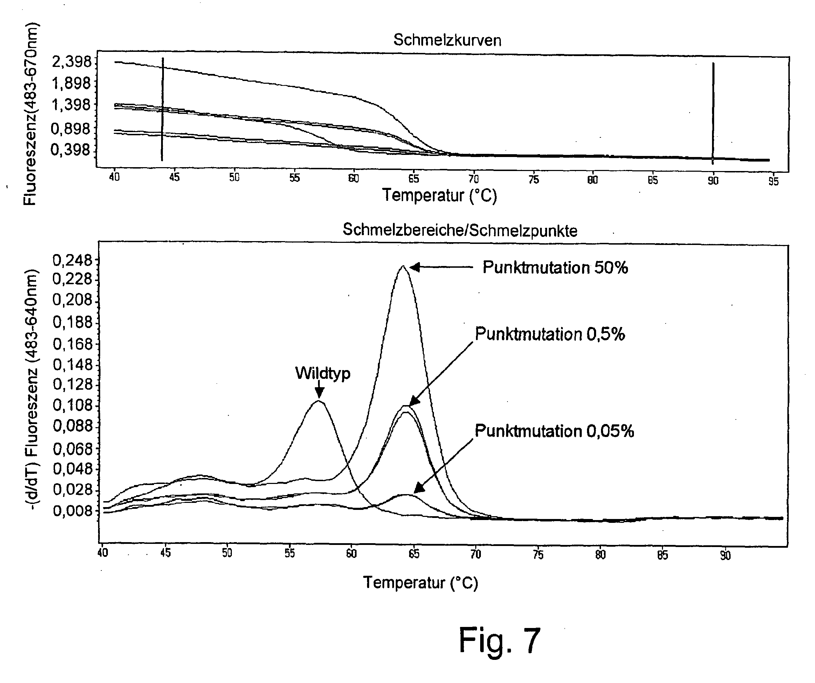

Schließlich zeigt Fig. 7 eine Darstellung der spezifischen Schmelzkurven der T790M-Mutation. Während der Schmelzkurvenanalyse wird die Abnahme der Fluoreszenz gemessen. Das Signal des Wildtyps (linkes Maximum) wird in Proben, die ausschließlich Wildtyp-DNA beinhalten, nahezu inhibiert. Proben mit der in Rede stehenden Punktmutation, unabhängig ob homozygot oder heterozygot, sind durch einen Peak bzw. ein Maximum mit höherer Schmelztemperatur nachzuweisen (rechte Maxima.) Proben, die nur einen minimalen Gehalt an mutierter DNA enthalten, weisen ebenfalls eine Kurve bei der spezifischen Schmelztemperatur der Mutanten auf. In bezug auf die T790M-Mutation kann ein Anteil von bereits 0,05 % T790M-mutierter Zellen, bezogen auf den Gesamtgehalt an Zellen in der Probe, in einem Wildtyp-Gemisch detektiert werden.

Die vorliegende Erfindung wird nachfolgend weiterführend und nichtbeschränkend veranschaulicht:

Im Zusammenhang mit der vorliegenden Erfindung konnte gezeigt werden, daß durch EGFR-Inhibitoren die Aktivierung des EGF-Rezeptors (EGFR) gehemmt werden kann. Über 80 % der Mutationen des EGF-Rezeptors bei NSCLC-Patienten beruhen auf verschiedenen Deletionen in Exon 19, sowie auf einer Punktmutation in Exon 2, nämlich L858R (Austausch der Aminosäure Leucin L an Position 858 durch Arginin R, vgl. Fig. 1). Patienten mit einem Lungentumor, welche eine dieser Veränderungen aufweisen, sind somit besonders geeignet für eine Therapie mit EGFR-Inhibitoren.

Obwohl die Therapie im allgemeinen gut vertragen wird und sehr spezifisch wirksam ist, entwickeln die meisten Patienten nach einer gewissen Zeit eine sogenannte Sekundärmutation, die zusätzlich zu der bereits vorhandenen Mutation auftreten kann und zur Resistenz gegenüber Erlotinib und Gefitinib führt. In etwa 50 bis 65% dieser Fälle findet man die Mutation T790M (Austausch der Aminosäure Threonin T durch Methionin M an Position 790, vgl. Fig. 1). Für diese Patienten stehen Medikamente zur Verfügung, deren Wirkmechanismus sich von denen der ersten Generation (Erlotinib, Gefitinib) unterscheidet. Diese Inhibitoren der so genannten zweiten Generation binden irreversibel an den Rezeptor und nicht reversibel, wie z.B. Tarceva^{®} oder Iressa^{™}. NSCLC-Patienten, die aufgrund der T790M-Mutation nicht mehr auf Medikamente der ersten Generation ansprechen, können folglich mit einem EGFR-Inhibitor der zweiten Generation (z.B. BIBW2992/Tovok von Boehringer Ingelheim) weiterbehandelt werden. Wie klinische Studien zeigen, sind diese Inhibitoren ebenfalls hochspezifisch und wirksam, so daß das Wachstum und Überleben der Tumorzellen verlangsamt oder verhindert werden kann.

Im Sinne einer individualisierten Medizin, also um jedem einzelnen Patienten mit NSCLC die für ihn am besten geeignete Therapie bieten zu können, ist es notwendig und sinnvoll, das Tumorgewebe auf den EGFR-Status zu testen. Somit kann denjenigen Patienten, die eine aktivierende Mutation im EGF-Rezeptor aufweisen, eine Behandlung mit den entsprechenden EGFR-Inhibitoren angeboten werden.

Um einen hohen Grad an Sensitivität zu erreichen, werden im Rahmen der vorliegenden Erfindung verschiedene Optimierungsschritte realisiert, auf die am Beispiel der Punktmutation L858R im nachfolgenden veranschaulichend eingegangen wird.

Zu Beginn der Planung bzw. Durchführung des Mutationsnachweises werden zunächst spezifische Primer und Hybridisierungssonden generiert bzw. bereitgestellt. Derartige Primer und Hybridisierungssonden können beispielsweise von der Firma TIB MOLBIOL GmbH, Berlin, bezogen werden.

Die spezifischen Primer amplifizieren bevorzugt den Abschnitt der DNA, in dem die nachzuweisende Mutation lokalisiert ist. Zusätzlich werden in der PCR-Reaktion gemäß einer bevorzugten Ausführungsform zwei voneinander verschiedene Hybridisierungssonden (Sensorsonde einerseits und Ankersonde andererseits) eingesetzt. Diese umfassen, wie die Primer auch, mehrere Nukleotide, die innerhalb des entstehenden PCR-Produktes hybridisieren. Dabei binden beide Sonden in räumlicher Nähe zueinander. An den einander zugewandten Enden sind die Sonden jeweils mit einem Fluoreszenzfarbstoff markiert und können über FRET (Fluoreszenz-Resonanz-Energie-Transfer) miteinander interagieren (vgl. Fig. 2). Die Sonden binden sowohl in bzw. an der Wildtypsequenz (wildtypischer DNA-Einzelstrang bzw. wildtypischer DNA-Strang) als auch an der Mutationssequenz (mutierter DNA-Einzelstrang bzw. mutierter DNA-Strang), wobei eine der Sonden (Ankersonde) im nicht veränderten Sequenzbereich und die andere Sonde (Sensorsonde) im Bereich der vermuteten (Punkt-)Mutation bindet.

Sofern eine Punktmutation an der Bindungsstelle vorliegt und sofern die Sensorsonde passend zur mutierten Sequenz synthetisiert wurde, bindet diese Sonde spezifisch. Sofern keine Punktmutation vorliegt, bindet die Sensorsonde entsprechend unspezifisch. In beiden Fällen überträgt die Sensorsonde unter bzw. nach energetischer Anregung mit elektromagnetischer Strahlung einer entsprechenden Wellenlänge, wie sie durch die Xenon-Lampe des Light-Cyclers^{®} erzeugt werden kann, ihre Energie durch FRET auf die Ankersonde. Diese wird hierdurch ebenfalls angeregt und emittiert ein Fluoreszenzsignal, welches vom Gerät detektiert werden kann. Die Messung kann am Ende der *Annealing*-Phase jedes PCR-Zyklus, der Phase, in welcher die Primer und Sonden an den DNA-Strang binden, durchgeführt werden.

Im Anschluß an die eigentliche PCR schließt sich eine Schmelzkurvenanalyse an. Hier werden die Reaktionsansätze langsam beispielsweise auf 95°C erhitzt. An dem Punkt, an dem sich 50 % der Sensorsonden von den entstandenen PCR-Produkten gelöst haben, fällt die Fluoreszenz drastisch ab. Bindet die Sonde spezifisch an den mutierten Strang, erfolgt die Dehybridisierung bzw. Ablösung der Sensorsonde bei einer höheren Temperatur im Vergleich zu einer Bindung an Wildtyp-Sequenzen. Zusätzlich zur Abnahme des Fluoreszenzsignals mit steigender Temperatur wird insbesondere unter Verwendung des LightCycler^{®} 480 die erste mathematische Ableitung der Schnittkurve berechnet, so daß der Wendepunkt der gemessenen Fluoreszenzabnahme als Kurve dargestellt wird. Der höchste Punkt bzw. das Maximum der Kurve auf Basis der ersten Ableitung entspricht der spezifischen Schmelztemperatur. Diese Darstellung dient der besseren visuellen Auswertung. Heterozygote Proben, also Zellen, die sowohl ein wildtypisches als auch ein mutiertes Allel besitzen, zeigen folglich zwei verschiedene Schmelzprofile, die entsprechend als "Doppelpeak" bzw. Doppelmaximum (Ausbildung zweier unterschiedlicher Maxima) zu sehen sind (vgl. Fig. 3).

Nach etwaiger Optimierung der eingesetzten DNA-Menge, der *Annealing-*Temperatur, der Primerkompatibilität und/oder -konzentration sowie der Sondenkonzentration zeigt der Nachweis der L858R-Mutation unter Bedingungen des Standes der Technik im Rahmen einer üblichen und nichtasymmetrischen PCR genauso wie im Falle einer Sequenzierung eine Sensitivität von lediglich 20 bis 25%.

Um die Nachweisgrenze zu steigern, wird im Rahmen der vorliegenden Erfindung in zweckgerichteter Weise eine sogenannte asymmetrische PCR angewendet bzw. durchgeführt. Im Gegensatz zur symmetrischen PCR ist hierbei das Verhältnis beider Primer zueinander nicht gleich. Es wird die Konzentration desjenigen Primers in dem PCR-Ansatz erhöht, welcher an denjenigen DNA-Strang bindet, an dem auch die Sonden hybridisieren (Sondenstrang). Somit wird dieser Strang bevorzugt vervielfältigt und die eingesetzten Sonden haben damit eine höhere Wahrscheinlichkeit, an diesen Strang zu binden (vgl. Fig. 3). Auf diese Weise kann auch die Sensitivität des Mutationsnachweises erhöht werden.

Zur weiteren Steigerung der Sensitivität ist es erfindungsgemäß zudem vorgesehen, die Amplifikation des Wildtyp-DNA-Strangs und/oder die Bindung der Sensorsonde an den Wildtyp-DNA-Strangs zu verringern bzw. zu inhibieren, so daß nur bzw. vorrangig der mutierte DNA-Strang vervielfältigt wird und die eingesetzten Sonden somit gewissermaßen konkurrenzlos an die mutierte DNA binden können. Mit Hilfe dieser erfindungsgemäßen Technik können in völlig überraschender Weise selbst kleinste Mengen mutierten Materials nachgewiesen werden. Diese spezifische Hemmung des Wildtyps kann z. B. mit einem Blockierungsmittel insbesondere in Form einer sogenannten *Locked Nucleic Acid* (LNA^{™} von der Firma Exiqon), welche auch *"Clamp"* genannt wird, durchgeführt werden (vgl. Fig. 4). Diese "*Clamps*" sind biochemisch so modifiziert, daß sie eine sehr hohe Bindungsstärke gegenüber dem Wildtypstrang aufweisen und erst bei sehr hohen Temperaturen abschmelzen.

Während der Schmelzkurvenanalyse wird die Abnahme der Fluoreszenz gemessen. Ist in der Probe eine Mutation nachzuweisen, ist die entsprechende Kurve sichtbar; sofern kein mutiertes Material vorhanden ist, ist keine bzw. eine minimale Wildtyp-Kurve zu sehen, da aufgrund der fehlenden Amplifikation und Sondenbindung des Wildtyps kein Signal gemessen werden kann (vgl. Abb. 6 und Abb. 7).

Um bestätigen zu können, daß die PCR-Reaktion erfolgreich durchgeführt wurde und um Heterozygotien nachweisen zu können, sollte auch ein Vergleichsansatz ohne Blockierungsmittel mitgeführt bzw. durchgeführt werden. In dieser Reaktion sollte zumindest der Wildtyp sichtbar sein.

Die erfindungsgemäße Kombination der oben genannten Maßnahmen ermöglicht Mutationsnachweise mit äußerst hoher Sensitivität. Im genannten Beispiel des Nachweises der L858R-Mutation kann eine Sensitivität von 0,0005 % mutierter DNA in einem Wildtyp-Gemisch realisiert werden. Somit können kleinste Anteile mutierter DNA beispielsweise eines Tumorgewebes in einer Probe nachgewiesen werden. Auch eignet sich das erfindungsgemäße Verfahren, um Verlaufskontrollen im peripheren Blut oder anderen Körperflüssigkeiten durchzuführen. Bei vielen Patienten mit soliden Tumoren, wie z. B. NSCLC-Patienten wird meist nur einmal Tumormaterial gewonnen, z. B. bei der Erstdiagnose oder einer Operation. Somit ist das *Monitoring* bzw. die Beobachtung des Erkrankungsverlaufs auf molekularer Ebene meist nicht möglich. Mit dem erfindungsgemäßen hochsensitiven Nachweisverfahren, welches auch in Kombination mit sensitiven Aufreinigungsmethoden durchgefiihrt werden kann, können sogar zirkulierende Tumorzellen in einer Vollblutprobe nachgewiesen werden. Es wurde beispielsweise beschrieben, daß Patienten mit NSCLC etwa 100 bis 200 zirkulierende Tumorzellen pro 1 ml Blut aufweisen. Geht man von etwa 10.000 kernhaltigen Zellen pro µl aus, bedeutet das einen Gehalt von etwa 0,001 % Tumorzellen. Mit der erfindungsgemäßen Real-Time-PCR-Methode, die auch ein sensitives Aufreinigungsverfahren der Tumorzellen umfassen kann, ist dieser Nachweis und damit eine Verlaufskontrolle bei Patienten vor bzw. unter Therapie möglich. Die vorliegende Erfindung stellt somit einen wichtigen Schritt in Richtung einer individualisierten und damit hochspezifischen Therapie dar.

Weitere Ausgestaltungen, Abwandlungen und Variationen der vorliegenden Erfindung sind für den Fachmann beim Lesen der Beschreibung ohne weiteres erkennbar und realisierbar, ohne daß er dabei den Rahmen der vorliegenden Erfindung verläßt.

Das nachfolgende Ausführungsbeispiel dient lediglich zur Veranschaulichung der vorliegenden Erfindung, ohne die vorliegende Erfindung jedoch hierauf zu beschränken.

### AUSFÜHRUNGSBEISPIEL:

### EGFR-T790M-Mutationsnachweis:

Nachweis der Mutation T790M in genomischer DNA mittels Schmelzkurvenanalyse am LightCycler^{®} 480:

### Zusätzliche Referenzunterlagen:

- Datenblatt bzw. Data Sheet "LightCycler 480 Probes Master" von F. Hoffmann-La Roche Ltd.;
- Datenblatt bzw. Data Sheet "PureLink Genomic DNA Mini Kit" von Invitrogen Corp.

### Material:

- *LightCycler^{®} 480 Probes Master Kit* von F. Hoffmann-La Roche Ltd. (Best.-Nr. 04887301001);
- Primer und Sonden von TIB MOLBIOL GmbH, Berlin:
   Primer: EGFR F (Prod.-Nr: 1030632) und EGFR A (Prod.-Nr. 1030635);
   Sonden: Anchor_790 (Prod.-Nr. 1030640) und Sensor [T] (Prod.-Nr. 1030639) und 790 LNA wt (Prod.-Nr. 1084404);
- genomische DNA , z. B. aus peripherem Blut (Citrat);
   (20 - 90 µg/ml Ausgangskonzentration);
   isoliert mit "PureLink Genomic DNA Mini Kit" von Invitrogen Corp. (Best.-Nr. K1820-01);
- Kontroll-DNA: z. B. A431 (EGFR Wildtyp) und H1975 (EGFR L858R Mutante);
- 96-Well-Platten von F. Hoffmann-La Roche Ltd., weiß (*LightCycler 480-Multiwell-Plate 96,* Best.-Nr. 04729692001)

### Geräte:

- Zentrifuge mit Einsatz für 96-well-Platten (z.B. Multifuge 3SR+ von Thermo Fisher Scientific Inc.);
- LightCycler^{®} 480 von F. Hoffmann-La Roche Ltd. mit 96-well-Block.

### Beschreibung des Ablaufs:

Auftauen der benötigten Kit-Komponenten, der Primer und der Sonden auf Eis (Sonden im Dunkeln auftauen)

### Pipettieren des Primer/Sonden-Mixes auf Eis:

| | | |
|---|---|---|
| EGFR F: | 0,4µl (8 pmol) | 20 pmol/µl |
| + EGFR A: | 0, 06 µl (1, 2 pmol) | 20 pmol/µl |
| + Anchor_790: | 0, 15 µl (3 pmol) | 20 pmol/µl |
| + Sensor [T]: | 0,15 µl (3 pmol) | 20 pmol/µl |
| + 790 LNA wt: | 0,275 µl (0, 55 pmol) | 2 pmol/µl |
| + PCR-reines Wasser: | 0,965 µl | |

Gesamtvolumen: 2 µl

### Pipettieren des Mastermixes:

| | |
|---|---|
| Primer/Sonden-Mix gemäß obigen Ausführungen: | 2 µl |
| + 480-Probes-Master: | 10 µl |
| + PCR-reines Wasser: | 7,5 µl |
| Gesamtvolumen: | 19,5 µl |

In eine weiße 96-Well-Platte werden 19,5 µl Mastermix und 0,5 µl DNA pipettiert. Mitführen einer Wasserkontrolle ohne DNA, einer Negativkontrolle (Wildtyp, z. B. A431) und einer Positivkontrolle (Mutante, z.B. H1975). Zudem Mitführen eines Ansatzes ohne LNA-Sonde (entsprechender Anteil an Wasser im Primer/Sonden-Mix).

Abdichten der Platte mit Folie und Zentrifugation: 1500 g, 2 min bei RT

### Platte in LightCycler^{®} 480 einlegen und Programm starten:

| Filter-Kombinationen: | Schmelzfaktor: | Quantfaktor: | Max. Integrationszeit: |
|---|---|---|---|
| 483-640 | 1,2 | 5 | 2 Sekunden |

| Ablaufprotokoll: | Temperatur | Zeit | Zyklen |
|---|---|---|---|
| 1. Präinkubation: | 95 °C | 00:10:00 | 1 |
| 2. Amplifikation: | 95 °C | 00:00:10 | 40 |
| | 56 °C | 00:00:10 | |
| | 72 °C | 00:00:10 | |
| 3. Schmelzkurve: | 95 °C | 00:01:00 | 1 |
| | 40 °C | 00:02:00 | |
| | 95 °C | kontinuierlich | |
| 4. Abkühlung | 4 °C | kontinuierlich | |

### Erwartetes Ergebnis der Schmelzkurvenanalyse:

| Ergebnis: | Schmelzkurventemperaturen |
|---|---|
| Sonden binden spezifisch an Mutante | |
| homozygote Mutante: | eine Schmelzkurve zwischen 55 und 70 °C |
| homozygoter Wildtyp: | eine Schmelzkurve zwischen 45 und 60 °C |
| Heterozygotie: | zwei Schmelzkurven; s.o. |

Hinweise und Anmerkungen: Der fertige PCR-Mix ist zumindest 24 Stunden bei RT im Dunkeln stabil.

| | |
|---|---|
| Abkürzung: | RT = Raumtemperatur |

### Sequenzprotokoll I

| | | |
|---|---|---|
| LOCUS | NP_005219 1210 aa linear PRI | |
| 05-OCT-2009 | | |
| DEFINITION | epidermal growth factor receptor isoform a precursor | |
| [Homo sapiens]. | | |
| ACCESSION | NP_005219 | |
| VERSION | NP_005219.2 GI:29725609 | |
| DBSOURCE | REFSEQ: accession NM 005228.3 | |
| KEYWORDS | . | |
| SOURCE | Homo sapiens (human) | |
| ORGANISM | Homo sapiens | |
| | Eukaryota; Metazoa; Chordata; Craniata; Vertebrata; Euteleostomi; Mammalia; Eutheria; Euarchontoglires; Primates; Haplorrhini; Catarrhini; Hominidae; Homo. | |
| REFERENCE | 1 (residues 1 to 1210) | |
| AUTHORS | Donati,V., Lupi,C., Ali,G., Corsi,V., Viti,A., Lucchi,M., Mussi,A.and Fontanini,G. | |
| TITLE | Laser capture microdissection: a tool for the molecular characterization of histologic subtypes of lung adeno carcinoma | |
| JOURNAL | Int. J. Mol. Med. 24 (4), 473-479 (2009) | |
| PUBMED | 19724887 | |
| REMARK | GeneRIF: Observational study of gene-disease association. (HuGE | |
| | Navigator) | |
| REFERENCE | 2 (residues 1 to 1210) | |
| AUTHORS | Boldrini,L., Ali,G., Gisfredi,S., Ursino,S., Baldini,E., Melfi,F. , | |
| | Lucchi,M., Comin,C.E., Maddau,C., Tibaldi,C., Camacci,T., | |
| | Servadio,A., Mussi,A. and Fontanini,G. | |
| TITLE | Epidermal growth factor receptor and K-RAS mutations in 411 lung | |
| | adenocarcinoma: A population-based prospective study | |
| JOURNAL | Oncol. Rep. 22 (4), 683-691 (2009) | |
| PUBMED | 19724844 | |
| REMARK | GeneRIF: Observational study of gene-disease associati on. (HuGE Navigator) | |
| REFERENCE | 3 (residues 1 to 1210) | |
| AUTHORS | Krishnaswamy,S., Kanteti,R., Duke-Cohan,J.S., Loganathan,S., | |
| | Liu,W., Ma,P.C., Sattler,M., Singleton,P.A., Ramnath,N., Innocenti,F., Nicolae,D.L., Ouyang,Z., Liang,J., Minna,J., | |
| | Kozloff,M.F., Ferguson,M.K., Natarajan,V., Wang,Y.C., Garcia,J.G., | |
| | Vokes,E.E. and Salgia,R. | |
| TITLE | Ethnic differences and functional analysis of MET mutations in lung | |
| | cancer | |
| JOURNAL | Clin. Cancer Res. 15 (18), 5714-5723 (2009) | |
| PUBMED | 19723643 | |
| REMARK | GeneRIF: Observational study of gene-disease associati- | |
| | on. (HuGE | |
| | Navigator) | |
| REFERENCE | 4 (residues 1 to 1210) | |
| AUTHORS | Monaco,S.E., Nikiforova,M.N., Cieply,K., Teot,L.A., Khalbuss,W.E. | |
| | and Dacic,S. | |
| TITLE | A comparison of EGFR and KRAS status in primary lung carcinoma and | |
| | matched metastases | |
| JOURNAL | Hum. Pathol. (2009) In press | |
| PUBMED | 19740513 | |
| REMARK | GeneRIF: Observational study of gene-disease association. (HuGE Navigator) | |
| | Publication Status: Available-Online prior to print | |
| REFERENCE | 5 (residues 1 to 1210) | |
| AUTHORS | Hama,T., Yuza,Y., Saito,Y., O-uchi,J., Kondo,S., Okabe,M., | |
| | Yamada,H., Kato,T., Moriyama,H., Kurihara,S. and Urashima,M. | |
| TITLE | Prognostic significance of epidermal growth factor receptor | |
| | phosphorylation and mutation in head and neck squamous cell | |
| | carcinome | |
| JOURNAL | Oncologist 14 (9), 900-908 (2009) | |
| PUBMED | 19726454 | |
| REMARK | GeneRIF: Observational study of gene-disease association. (HuGE Navigator) | |
| REFERENCE | 6 (residues 1 to 1210) | |
| AUTHORS | Li,W., Hu,P., Skolnik,E.Y., Ullrich,A. and Schlessinger,J. | |
| TITLE | The SH2 and SH3 domain-containing Nck protein is oncogenic and a | |
| | common target for phosphorylation by different surface receptors | |
| JOURNAL | Mol. Cell. Biol. 12 (12), 5824-5833 (1992) | |
| PUBMED | 1333047 | |
| REFERENCE | 7 (residues 1 to 1210) | |
| AUTHORS | Krieg,J. and Hunter,T. | |
| TITLE | Identification of the two major epidermal growth factorinduced | |
| | tyrosine phosphorylation sites in the microvillar core protein | |
| | ezrin | |
| JOURNAL | J. Biol. Chem. 267 (27), 19258-19265 (1992) | |
| PUBMED | 1382070 | |
| REFERENCE | 8 (residues 1 to 1210) | |
| AUTHORS | Lowenstein,E.J., Daly,R.J., Batzer,A.G., Li,W., Margolis,B., | |
| | Lammers,R., Ullrich,A., Skolnik,E.Y., Bar-Sagi,D. and Schlessinger,J. | |
| TITLE | The SH2 and SH3 domain-containing protein GRB2 links receptor | |
| | tyrosine kinases to ras signaling | |
| JOURNAL | Cell 70 (3), 431-442 (1992) | |
| PUBMED | 1322798 | |
| REFERENCE | 9 (residues 1 to 1210) | |
| AUTHORS | Chi,D.D., Hing,A.V., Helms,C., Steinbrueck,T., Mishra,S.K. and | |
| | Donis-Keller,H. | |
| TITLE | Two chromosome 7 dinucleotide repeat polymorphisms at gene loci | |
| | epidermal growth factor receptor (EGFR) and pro alpha 2 | |
| | (I) collagen (COL1A2) | |
| JOURNAL | Hum. Mol. Genet. 1 (2), 135 (1992) | |
| PUBMED | 1301150 | |
| REFERENCE | 10 (residues 1 to 1210) | |
| AUTHORS | Countaway,J.L., Nairn,A.C. and Davis,R.J. | |
| TITLE | Mechanism of desensitization of the epidermal growth factor | |
| | receptor protein-tyrosine kinase | |
| JOURNAL | J. Biol. Chem. 267 (2), 1129-1140 (1992) | |
| PUBMED | 1309762 | |
| COMMENT | REVIEWED REFSEQ: This record has been curated by NCBI staff. The | |
| | reference sequence was derived from AW163038.1, X00588.1, | |
| | AF125253.1, AU137334.1, CB160831.1, AL598260.1, AI217671.1 and | |
| | AW295229.1. | |
| | On Apr 10, 2003 this sequence version replaced gi:4885199. | |
| Summary: | The protein encoded by this gene is a transmembrane glycoprotein that is a member of the protein kinase superfamily. | |
| | This protein is a receptor for members of the epidermal growth | |
| | factor family. EGFR is a cell surface protein that binds to | |
| | epidermal growth factor. Binding of the protein to a ligand induces | |
| | receptor dimerization and tyrosine autophosphorylation and leads to | |
| | cell proliferation. Mutations in this gene are associated with lung | |
| | cancer. [provided by RefSeq]. | |
| | Transcript Variant: This variant (1) is the longest tränscript and | |
| | it encodes the longest isoform (a). | |
| | Publication Note: This RefSeq record includes a subset of the | |
| | publications that are available for this gene. Please see the | |
| | Entrez Gene record to access additional publications. | |
| FEATURES | | Location/Qualifiers |
| source | | 1..1210 |
| | | /organism="Homo sapiens" |
| | | /db_xref="taxon:9606" |
| | | /chromosome="7" |
| | | /map="7p12" |
| Protein | | 1..1210 |
| | /product="epidermal growth factor receptor isoform a | |
| | precursor" | |
| | /EC_number="2.7.10.1" | |
| | /note="avian erythroblastic leukemia viral (v-erb-b) | |
| | oncogene homolog; cell growth inhibiting protein 40; cell | |
| | proliferation-inducing protein 61" | |
| sig peptide | 1..24 | |
| | | /calculated_mol_wt=2283 |
| mat peptide | 25..1210 | |
| | /product="epidermal growth factor receptor isoform a" | |
| | /calculated_mol_wt=132013 | |
| Region | 57..168 | |
| | /region_name="Recep_L_domain" | |
| | /note="Receptor L domain; pfam01030" | |
| | /db_xref="CDD:110059" | |
| Region | 185..337 | |
| | /region_name="Furin-like" | |
| | /note="Furin-like cysteine rich region; pfam00757" | |
| | /db_xref="CDD:109800" | |
| Region | 231..274 | |
| | /region_name="FU" | |
| | /note="Furin-like repeats. Cysteine rich region. Exact | |
| | function of the domain is not known. Furin is a serine-kinase dependent proprotein processor. Other | |
| | members of this family include endoproteases and cell | |
| | surface receptors; cd00064" | |
| | /db_xref="CDD:28946" | |
| Region | 361..481 | |
| | /region_name="Recep_L_domain" | |
| | /note="Receptor L domain; pfam01030" | |
| | /db_xref="CDD:110059" | |
| Region | 497..>598 | |
| | /region_name="Furin-like" | |
| | /note="Furin-like cysteine rich region; pfam00757" | |
| | /db_xref="CDD:109800" | |
| Region | 552..598 | |
| | /region_name="FU" | |
| | /note="Furin-like repeats. Cysteine rich region. Exact | |
| | function'of the domain is not known. Furin is a serine-kinase dependent proprotein processor. Other | |
| | members of this family include endoproteases and cell | |
| | surface receptors; c100066" | |
| | /db_xref="CDD:140407" | |
| Region | 704..1019 | |
| | /region_name="PTKc_EGFR" | |
| | /note="Catalytic Domain of the Protein Tyrosine Kinase, | |
| | Epidermal Growth Factor Receptor; cd05108" | |
| | /db_xref="CDD:133239" | |
| Region | 712..968 | |
| | /region_name="Pkinase_Tyr" | |
| | /note="Protein tyrosine kinase; pfam07714" | |
| | /db_xref="CDD:116328" | |
| Site | or- | |
| | der(715..717,728..730,794..795,797,804..805,100 9..1010) | |
| | /site_type="other" | |
| | /note="dimer interface" | |
| | /db_xref="CDD:133239" | |
| Site | or- | |
| | der(718..719,722..723,745,791,793,797,841..842, 855, | |
| | 876..880,885,889) | |
| | /site_type="active" | |
| | /db_xref="CDD:133239" | |
| Site | or- | |
| | der(718..719,726,743,745,766,790..791,793,841.. 842,844, | |
| | 855) | |
| | /site_type="other" | |
| | /note="ATP binding site" | |
| | /db_xref="CDD:133239" | |
| Site | 854..875 | |
| | /site_type="other" | |
| | /note="activation loop (A-loop)" | |
| | /db_xref="CDD:133239" | |
| Site | order(876..880,885,889) | |
| | /site type="other" | |
| | /note="substrate binding site" | |
| | /db_xref="CDD:133239" | |
| CDS | 1..1210 | |
| | /gene="EGFR" | |
| | /gene_synonym="ERBB; ERBB1; HER1; mENA; PIG61" | |
| | /coded_by="NM_005228.3:247..3879" | |
| | /note="isoform a precursor is encoded by transcript | |
| | variant 1" | |
| | /db_xref="CCDS:CCDS5514.1" | |
| | /db_xref="GeneID:1956" | |
| | /db_xref="HGNC:3236" | |
| | /db_xref="HPRD:00579" | |
| | /db_xref="MIM:131550" | |

### ORIGIN

## Patentansprüche

1. Verfahren zur Detektion mindestens einer Mutation in einem Gen, vorzugsweise in einem für ein mit einer Tumor- und/oder Krebserkrankung in Verbindung stehenden Protein kodierenden Gen, wobei das die Genveränderung aufweisende Gen (Mutationsgen) zusammen mit weiteren, für das Protein kodierenden, jedoch keine Mutation aufweisenden Genen (Wildtypgenen) vorliegt,
wobei das Verfahren mittels asymmetrischer Polymerase-Kettenreaktion *(Polymerase Chain Reaction* bzw. PCR) unter kombinierter Verwendung mindestens einer detektierbaren mutationspezifischen Hybridisierungssonde (Sensorsonde) einerseits und mindestens eines die Anbindung der Sensorsonde an die Wildtypgene inhibierenden wildtypspezifischen Blockierungsmittels andererseits durchgeführt wird,
wobei das Blockierungsmittel ausgewählt wird derart, daß das Blockierungsmittel eine höhere Spezifität in bezug auf den Bereich des DNA-Einzelstrangs des Wildtypgens, welcher zu dem die Mutation aufweisenden Genabschnitt des Mutationsgens korrespondiert, gegenüber dem entsprechenden Mutationsgen aufweist.

2. Verfahren nach Anspruch 1, wobei das Verfahren derart durchgeführt wird, daß eine selektive Verstärkung der Detektion in bezug auf einen die Mutation aufweisenden Genabschnitt des Mutationsgens erfolgt.

3. Verfahren nach Anspruch 1 oder 2, **dadurch gekennzeichnet,**
**daß** es sich bei der Tumor- und/oder Krebserkrankung um ein Lungenkarzinom, insbesondere um ein nichtkleinzelliges Lungenkarzinom *(Non Small Cell Lung Cancer* bzw. NSCLC) oder um ein kleinzelliges Lungenkarzinom (*Small Cell Lung Cancer* bzw. SCLC), vorzugsweise um ein nichtkleinzelliges Lungenkarzinom (NSCLC), handelt
und/oder daß es sich bei dem Protein um ein insbesondere humanes und/oder ein das Zellwachstum und/oder die Proliferation von Zellen regulierendes oder induzierendes Protein handelt
und/oder daß es sich bei dem Protein um einen transmembranen Rezeptor für Wachstumsfaktoren mit intrinsischer Tyrosinkinase-Aktivität handelt
und/oder daß es sich bei dem Protein um einen Epidermalen-Wachstumsfaktor-Rezeptor (*Epidermal-Growth-Factor*-Rezeptor bzw. EGF-Rezeptor) handelt.

4. Verfahren nach einem der vorangehenden Ansprüche, **dadurch gekennzeichnet,**
**daß** die Mutation zu einer Deletion in Exon 19 des EGF-Rezeptors führt und/oder
**daß** die Mutation zu einem Austausch mindestens einer Aminosäure in Exon 20 und/oder in Exon 21 des EGF-Rezeptors führt und/oder
**daß** die Mutation zu einem Austausch von Serin an Position 768 des EGF-Rezeptors, insbesondere durch Isoleucin (S768I), führt und/oder
**daß** die Mutation zu einem Austausch von Threonin an Position 790 des EGF-Rezeptors, insbesondere durch Methionin (T790M), führt und/oder daß die Mutation zu einem Austausch von Leucin an Position 858 des EGF-Rezeptors,
insbesondere durch Arginin (L858R), führt.

5. Verfahren nach einem der vorangehenden Ansprüche, **dadurch gekennzeichnet,**
**daß** eine Amplifizierung des der Deletion in Exon 19 des EGF-Rezeptors entsprechenden Genabschnitts des Mutationsgens erfolgt und/oder
**daß** eine Amplifizierung des dem Austausch von Serin an Position 768 des EGF-Rezeptors, insbesondere durch Isoleucin (S768I), entsprechenden Genabschnitts des Mutationsgens erfolgt und/oder
**daß** eine Amplifizierung des dem Austausch von Threonin an Position 790 des EGF-Rezeptors, insbesondere durch Methionin (T790M), entsprechenden Genabschnitts des Mutationsgens erfolgt und/oder
**daß** eine Amplifizierung des dem Austausch von Leucin an Position 858 des EGF-Rezeptors, insbesondere durch Arginin (L858R), entsprechenden Genabschnitts des Mutationsgens erfolgt und/oder
**daß** eine Amplifizierung des zu dem die Mutation aufweisenden Genabschnitt des Mutationsgens korrespondierenden Genabschnitts der Wildtypgene erfolgt.

6. Verfahren nach einem der vorangehenden Ansprüche, **dadurch gekennzeichnet, daß** ein erster Primer und ein hiervon verschiedener zweiter Primer eingesetzt werden.

7. Verfahren nach Anspruch 6, **dadurch gekennzeichnet,**
**daß** der erste Primer spezifisch an dem DNA-Einzelstrang des Mutationsgens (Sondenstrang) bindet, mit welchem die Sensorsonde zu interagieren imstande ist, und/oder
**daß** der zweite Primer spezifisch an dem zum Sondenstrang komplementären DNA-Einzelstrang des Mutationsgens (Komplementärstrang) bindet und/oder
**daß** der erste Primer und der zweite Primer ausgewählt werden derart, daß die Menge des ersten Primers, insbesondere bezogen auf den PCR-Ansatz, größer ist als die Menge des zweiten Primers, insbesondere so daß eine erhöhte und/oder verstärkte Amplifikation des Sondenstranges gegenüber dem Komplementärstrang erfolgt.

8. Verfahren nach einem der vorangehenden Ansprüche, **dadurch gekennzeichnet, daß** die Sensorsonde ausgewählt wird derart, daß die Sensorsonde eine höhere Spezifität in bezug auf den DNA-Einzelstrang des Mutationsgens (Sondenstrang), insbesondere im Bereich der Mutation gegenüber dem korrespondierenden DNA-Einzelstrang des Wildtypgens aufweist.

9. Verfahren nach einem der vorangehenden Ansprüche, **dadurch gekennzeichnet, daß** die Sensorsonde ausgewählt wird derart, daß die Sensorsonde im Falle der Wechselwirkung an den DNA-Einzelstrang des Mutationsgens (Sondenstrang) und/oder an den korrespondierenden DNA-Einzelstrang des Wildtypgens ein meßbares Signal, insbesondere Fluoreszenzsignal, abzugeben imstande ist

10. Verfahren nach einem der.vorangehenden Ansprüche, **dadurch gekennzeichnet, daß** die Sensorsonde im Falle einer insbesondere wärmeinduzierten Ablösung von dem DNA-Einzelstrang des Mutationsgens (Sondenstrang) und/oder von dem korrespondierenden DNA-Einzelstrang des Wildtypgens kein oder aber zumindest ein vermindertes meßbares Signal oder aber ein von dem angebundenen Zustand verschiedenes meßbares Signal abzugeben imstande ist.

11. Verfahren nach einem der vorangehenden Ansprüche, **dadurch gekennzeichnet, daß** zusätzlich zu der Sensorsonde mindestens eine hiervon verschiedene zweite Hybridisierungssonde (Ankersonde) eingesetzt wird.

12. Verfahren nach Anspruch 11, **dadurch gekennzeichnet, daß** die Sensorsonde einerseits und die Ankersonde andererseits ein FRET-Paar auszubilden imstande sind und/oder daß die Ankersonde ausgewählt wird derart, daß die Ankersonde auf demselben DNA-Einzelstrang wie die Sensorsonde zu binden imstande ist, insbesondere wobei die Ankersonde in einem Abstand von 1 bis 5 bp (Basenpaare) zu der Sensorsonde zu binden imstande ist.

13. Verfahren nach einem oder mehreren der vorangehenden Ansprüche, **dadurch gekennzeichnet,**
**daß** zur Detektion der Mutation, insbesondere im Anschluß an die Polymerase-Kettenreaktion, eine Schmelzkurve aufgenommen wird und/oder eine Schmelzkurvenanalyse durchgeführt wird und/oder
**daß** die Abspaltung insbesondere der Sensorsonde von dem jeweiligen DNA-Einzelstrang des Mutationsgens und/oder des Wildtypgens erfaßt wird, insbesondere wobei die Erfassung photometrisch, insbesondere durch Messung der Fluoreszenz, erfolgt, und/oder
**daß** über den oder die Schmelzpunkte der Schmelzkurve auf das Vorliegen einer Mutation geschlossen wird und im Falle des Vorliegens einer Mutation die Art der Mutation bestimmt wird.

14. Zusammensetzung, insbesondere zur Verwendung im Rahmen einer asymmetrischen Polymerase-Kettenreaktion *(Polymerase Chain Reaction* bzw. PCR) zur Detektion mindestens einer Mutation in einem Gen, vorzugsweise in einem für ein mit einer Tumor- und/oder Krebserkrankung in Verbindung stehenden Protein kodierenden Gen, wobei das die Mutation aufweisende Gen (Mutationsgen) zusammen mit weiteren, für das Protein kodierenden, jedoch keine Mutation aufweisenden Genen (Wildtypgenen) vorliegt,
wobei die Zusammensetzung in Kombination enthält:
(a) eine detektierbare mutationspezifische Hybridisierungssonde (Sensorsonde);
(b) einen ersten Primer, welcher zumindest im wesentlichen spezifisch an dem DNA-Einzelstrang des Mutationsgens (Sondenstrang), mit welchem die Sensorsonde zu interagieren imstande ist, bindet;
(c) einen zweiten Primer, welcher zumindest im wesentlichen spezifisch mit dem zum Sondenstrang komplementären DNA-Einzelstrang des Mutationsgens (Komplementärstrang) zu interagieren imstande ist;
(d) ein die Anbindung der Sensorsonde an die Wildtypgene inhibierendes wildtypspezifisches Blockierungsmittel;
wobei der Gehalt an dem ersten Primer (b) in der Zusammensetzung größer ist als der Gehalt an dem zweiten Primer (c).
wobei das Blockierungsmittel ausgewählt wird derart, daß das Blockierungsmittel eine höhere Spezifität in bezug auf den Bereich des DNA-Einzelstrangs des Wildtypgens, welcher zu dem die Mutation aufweisenden Genabschnitt des Mutationsgens korrespondiert, gegenüber dem entsprechenden Mutationsgen aufweist.

15. Verwendung der Zusammensetzung nach Anspruch 14 zur Detektion mindestens einer Mutation in einem Gen, vorzugsweise in einem für ein mit einer Tumor- und/oder Krebserkrankung in Verbindung stehenden Protein kodierenden Gen, wobei das die Mutation aufweisende Gen (Mutationsgen) zusammen mit weiteren, für das Protein kodierenden, jedoch keine Mutation aufweisenden Genen (Wildtypgenen) vorliegt, im Rahmen einer asymmetrischen Polymerase-Kettenreaktion *(Polymerase Chain Reaction* bzw. PCR).

## Claims

1. A method for the detection of at least one mutation in a gene, preferably in a gene coding for a protein in connection with a tumor and/or cancer illness, wherein the gene having the gene modification (mutation gene) is present together with additional genes (wild type genes) coding for the protein, but not having any mutations,
wherein the method is carried out by means of asymmetrical polymerase chain reaction (PCR) with the combined use of at least one detectable, mutation-specific hybridizing probe (sensor probe) on one hand, and at least one wild type-specific blocking agent inhibiting the binding of the sensor probe to the wild type gene,
wherein the blocking agent is selected such that the blocking agent has a higher specificity in terms of the area of the DNA single strand of the wild type gene, which corresponds to that gene section of the mutation gene having the mutation, as opposed to the respective mutation gene.

2. The method according to claim 1, wherein the method is carried out such that a selective amplification of the detection in terms of a gene section of the mutation gene having the mutation occurs.

3. The method according to claims 1 or 2, **characterized in that** the tumor and/or cancer illness is lung cancer, in particular non-small cell lung cancer (NSCLC), or small cell lung cancer (SCLC), preferably non-small cell lung cancer (NSCLC),
and/or that the protein is in particular a human protein, and/or one that regulates or induces the cell growth and/or the proliferation of cells,
and/or that the protein is a transmembrane receptor for growth factors with intrinsic tyrosinkinase activity,
and/or that the protein is an epidermal growth factor receptor (EGF receptor).

4. The method according to one of the previous claims, **characterized in that** the mutation leads to a deletion in Exon 19 of the EGF receptor, and/or that the mutation leads to an exchange of at least one amino acid in Exon 20, and/or in Exon 21 of the EGF receptor, and/or
the mutation leads to an exchange of serine at position 768 of the EGF receptor, in particular by means of isoleucine (S768I), and/or
the mutation leads to an exchange of threonine at position 790 of the EGF receptor, in particular by means of methionine (T790M), and/or
the mutation leads to an exchange of leucine at position 858 of the EGF receptor, in particular by means of arginine (L858R).

5. The method according to one of the previous claims, **characterized in that**
an amplification of the gene section of the mutation gene corresponding to the deletion in Exon 19 of the EGF receptor occurs, and/or
an amplification of the gene section of the mutation gene corresponding to the exchange of serine occurs at position 768 of the EGF receptor, in particular by means of isoleucine (S768I), and/or
an amplification of the gene section of the mutation gene corresponding to the exchange of threonine occurs at position 790 of the EGF receptor, in particular by means of methionine (T790M), and/or
an amplification of the gene section of the mutation gene corresponding to the exchange of leucine occurs at position 858 of the EGF receptor, in particular by means of arginine (L858R), and/or
an amplification of the gene section of the wild type gene corresponding to the gene section of the mutation gene having the mutation occurs.

6. The method according to one of the previous claims, **characterized in that** a first primer, and a second primer differing from the first, are utilized.

7. The method according to claim 6, **characterized in that**
the first primer specifically binds to the DNA single strand of the mutation gene (probe strand), with which the sensor probe is able to interact, and/or
the second primer specifically binds to the complementary DNA single strand of the mutation gene (complementary strand), and/or
the first primer, and the second primer, are selected such that the amount of the first primer, in particular based on the PCR approach, is greater than the amount of the second primer, in particular such that an increased and/or amplified amplification of the probe strand occurs, as opposed to the complementary strand.

8. The method according to one of the previous claims, **characterized in that** the sensor probe is selected such that the sensor probe has a higher specificity in terms of the DNA single strand of the mutation gene (probe strand), in particular in the area of the mutation, as opposed to the corresponding DNA single strand of the wild type gene.

9. The method according to one of the previous claims, **characterized in that** the sensor probe is selected such that the sensor probe is capable of giving off a measurable signal, in particular a fluorescence signal, to the DNA single strand of the mutation gene (probe strand), and/or to the corresponding DNA single strand of the wild type gene in case of the interaction.

10. The method according to one of the previous claims, **characterized in that** the sensor probe is capable of not giving off, or at least giving off a reduced measurable signal, or is capable of giving off a measurable signal that is different from the bound condition in case of particularly a heat induced separation from the DNA single strand of the mutation gene (probe strand), and/or from the corresponding DNA single strand of the wild type gene.

11. The method according to one of the previous claims, **characterized in that** in addition to the sensor probe, at least one of the second hybridization probes (anchoring probes) that differ from the same, is utilized.

12. The method according to claim 11, **characterized in that** the sensor probe on one hand, and the anchoring probe on the other hand, are each capable of embodying a FRET pair, and/or that the anchoring probe is selected such that the anchoring probe is capable of binding on the same DNA single strand as the sensor probe, in particular, wherein the anchoring probe is capable of binding to the sensor probe at a distance of 1 to 5 bp (base pairs).

13. The method according to one or more of the previous claims, **characterized in that**
for the detection of the mutation, in particular subsequent to the polymerase chain reaction, a melting curve is recorded, and/or a melting curve analysis is performed, and/or
the separation, in particular of the sensor probe, of the respective DNA single strand of the mutation gene, and/or of the wild type gene, is detected, in particular, wherein the detection is carried out photometrically, in particular by means of measuring the fluorescence, and/or
a conclusion on the presence of a mutation is made via the melting point(s) of the melting curve, and in case of the presence of a mutation, the type of mutation is determined.

14. A composition, in particular for the use within the scope of an asymmetrical polymerase chain reaction (PCR) for the detection of at least one mutation in a gene, preferably in a gene coding for a protein in connection with a tumor and/or cancer illness, wherein the gene comprising the mutation (mutation gene) is present together with further genes (wild type genes) coding for the protein, but having no mutations,
wherein the composition contains in combination:
(a) a detectable, mutation-specific hybridization probe (sensor probe);
(b) a first primer, which, at least essentially, specifically binds to the DNA single strand of the mutation gene (probe strand), with which the sensor probe is capable of interacting;
(c) a second primer, which, at least essentially, is specifically capable of interacting with the DNA single strand of the mutation gene that is complementary to the probe strand (complementary strand);
(d) a wild type specific blocking agent inhibiting the binding of the sensor probe to the wild type gene;
wherein the content at the first primer (b) in the composition is greater than the content at the second primer (c),
wherein the blocking agent is selected such that the blocking agent has a higher specificity in terms of the area of the DNA single strand of the wild type gene corresponding to the gene section of the mutation gene having the mutation, as opposed to the respective mutation gene.

15. A use of the composition according to claim 14 for the detection of at least one mutation in a gene, preferably in a gene coding for a protein in connection with a tumor and/or cancer illness, wherein the gene having the mutation (mutation gene) is present together with further genes (wild type genes) coding for the protein, but not having any mutations, within the scope of an asymmetrical polymerase chain reaction (PCR).

## Revendications

1. Procédé de détection d'au moins une mutation dans un gène, de préférence dans un gène codant une protéine, se trouvant en relation avec une affection tumorale et/ou cancéreuse, le gène présentant la modification génique (gène mutant) existant ensemble avec d'autres gènes codant pour la protéine mais ne présentant pas de mutation (gènes de type sauvage),
le procédé étant exécuté au moyen d'une réaction en chaîne asymétrique de polymérase (Polymerase Chain Reaction, c'est-à-dire PRC) et moyennant l'utilisation combinée d'au moins une sonde d'hybridation détectable spécifique de mutation (sonde capteur) d'une part et d'au moins un moyen de blocage spécifique du type sauvage, inhibant l'attache de la sonde capteur au gène de type sauvage d'autre part,
le moyen de blocage étant choisi de telle sorte que ledit moyen de blocage présente, en comparaison du gène mutant correspondant, une haute spécificité en rapport avec la zone du brin unitaire d'ADN du gène de type sauvage, laquelle correspond au tronçon du gène mutant présentant la mutation.

2. Procédé selon la revendication 1, le procédé étant exécuté de telle sorte qu'une amplification sélective de la détection s'effectue en rapport avec un tronçon de gène du gène mutant présentant la mutation.

3. Procédé selon la revendication 1 ou 2, **caractérisé en ce que**
il s'agit en matière d'affection tumorale et/ou cancéreuse d'un carcinome pulmonaire, en particulier d'un carcinome pulmonaire non à petites cellules (Non Small Cell Lung Cancer, c'est-à-dire NSCLC) ou d'un carcinome pulmonaire à petites cellules (Small Cell Lung Cancer, c'est-à-dire SCLC), de préférence d'un carcinome pulmonaire non à petites cellules (NSCLC),
et/ou **en ce qu'**en matière de protéine, il s'agit d'une protéine en particulier humaine et/ou d'une protéine régularisant ou induisant la croissance cellulaire et/ou la prolifération des cellules,
et/ou **en ce qu'**en matière de protéine, il s'agit d'un récepteur transmembranaire pour des facteurs de croissance à activité intrinsèque de tyrosine kinase et/ou **en ce qu'**en matière de protéine, il s'agit d'un récepteur de facteur de croissance épidermique (récepteur Epidermal-Groth-Factor, c'est-à-dire récepteur EGF).

4. Procédé selon l'une quelconque des revendications précédentes, **caractérisé en ce que**
la mutation conduit à une délétion dans l'exon 19 du récepteur EGF et/ou en ce que la mutation conduit à un échange d'au moins un acide aminé dans l'exon 20 et/ou dans l'exon 21 du récepteur EGF et/ou
**en ce que** la mutation conduit à un échange de la sérine en position 768 du récepteur EGF, en particulier par de l'isoleucine (S768I) et/ou
**en ce que** la mutation conduit à un échange de la thréonine en position 790 du récepteur EGF, en particulier par de la méthionine (T790M), et/ou
**en ce que** la mutation conduit à un échange de la leucine en position 858 du récepteur EGF, en particulier par de l'arginine (L858R).

5. Procédé selon l'une quelconque des revendications précédentes, **caractérisé en ce que**
il s'effectue une amplification du tronçon du gène mutant correspondant à la délétion dans l'exon 19 du récepteur EGF, et/ou
il s'effectue une amplification du tronçon du gène mutant correspondant à l'échange de la sérine en position 768 du récepteur EGF, en particulier par de l'isoleucine (S768I), et/ou
il s'effectue une amplification du tronçon du gène mutant correspondant à l'échange de la thréonine en position 790 du récepteur EGF, en particulier par de la méthionine (T790M), et/ou
il s'effectue une amplification du tronçon du gène mutant correspondant à l'échange de la leucine en position 858 du récepteur EGF, en particulier par de l'arginine (L858R), et/ou
il s'effectue une amplification du tronçon du gène de type sauvage, correspondant chez le gène mutant au tronçon de gène présentant la mutation.

6. Procédé selon l'une quelconque des revendications précédentes, **caractérisé en ce qu'**une première amorce et une deuxième amorce différente de celle-ci sont utilisées.

7. Procédé selon la revendication 6, **caractérisé en ce que** la première amorce se lie spécifiquement au brin unitaire d'ADN du gène mutant (brin sonde) avec lequel la sonde capteur est en mesure d'interagir, et/ou en ce que la deuxième amorce se lie spécifiquement au brin unitaire d'ADN du gène mutant complémentaire du brin sonde (brin complémentaire) et/ou en ce que la première amorce et la deuxième amorce sont choisies de telle sorte que la quantité de la première amorce, en particulier rapportée à la préparation PCR, est plus grande que la quantité de la deuxième amorce, en particulier de telle façon qu'il s'effectue une amplification accrue et/ou renforcée du brin sonde en comparaison du brin complémentaire.

8. Procédé selon l'une quelconque des revendications précédentes, **caractérisé en ce que** la sonde capteur est choisie de telle sorte que ladite sonde capteur présente une plus haute spécificité en rapport au brin unitaire d'ADN du gène mutant (brin sonde), en particulier dans la zone de la mutation, en comparaison du brin unitaire d'ADN correspond du gène de type sauvage.

9. Procédé selon l'une quelconque des revendications précédentes, **caractérisé en ce que**, dans le cas d'une interaction sur le brin unitaire d'ADN du gène mutant (brin sonde) et/ou sur le brin unitaire correspondant d'ADN du gène de type sauvage, la sonde capteur est en mesure d'émettre un signal mesurable, en particulier un signal de fluorescence.

10. Procédé selon l'une quelconque des revendications précédentes, **caractérisé en ce que**, dans le cas d'une séparation, en particulier induite par la chaleur, depuis le brin unitaire du gène mutant (brin sonde) et/ou depuis le brin unitaire correspondant d'ADN du gène de type sauvage, la sonde capteur n'est pas en mesure d'émettre un signal mesurable, ou n'est en mesure d'émettre tout au plus qu'un signal mesurable diminué, ou encore un signal mesurable différent de celui de l'état lié.

11. Procédé selon l'une quelconque des revendications précédentes, **caractérisé en ce qu'**en plus de la sonde de capteur, au moins une deuxième sonde d'hybridation (sonde ancre) différente de celle-ci est utilisée.

12. Procédé selon la revendication 11, **caractérisé en ce que** la sonde capteur d'une part et la sonde ancre d'autre part sont en mesure de former une paire de FRET et/ou **en ce que** la sonde ancre est choisie de telle sorte que ladite sonde ancre est en mesure de se lier sur le même brin unitaire d'ADN que la sonde capteur, en particulier **en ce que** la sonde ancre est en mesure de se lier à une distance de 1 à 5 bp (paires de bases) de la sonde capteur.

13. Procédé selon l'une ou plusieurs des revendications précédentes, **caractérisé en ce que**
pour la détection de la mutation, en particulier à l'issue de la réaction en chaîne de polymérase, une courbe de fusion est enregistrée et/ou une analyse de courbes de fusion est effectuée, et/ou
**en ce que** l'élimination, en particulier de la sonde capteur depuis le brin unitaire d'ADN respectif du gène mutant et/ou du gène de type sauvage, est détectée, en particulier **en ce que** la détection s'effectue photométriquement, en particulier par mesure de la fluorescence, et/ou
**en ce que**, par l'intermédiaire des points de fusion de la courbe de fusion, l'existence ou non d'une mutation est conclue et, dans le cas de la présence d'une mutation, le genre de mutation est déterminé.

14. Composition, en particulier pour une utilisation dans le cadre d'une réaction en chaîne asymétrique de polymérase (Polymerase Chain Reaction, c'est-à-dire PRC) pour la détection d'au moins une mutation dans un gène, de préférence dans un gène codant pour une protéine se trouvant en relation avec une affection tumorale et/ou cancéreuse, le gène présentant la modification génique (gène mutant) existant ensemble avec d'autres gènes codant pour la protéine mais ne présentant pas de mutation (gènes de type sauvage),
sachant que la composition contient en combinaison :
(a) une sonde d'hybridation (sonde capteur) détectable spécifique de mutation ;
(b) une première amorce, laquelle se lie de façon au moins essentiellement spécifique au brin d'ADN unitaire du gène mutant (brin sonde) avec lequel la sonde capteur est en mesure d'interagir ;
(c) une deuxième amorce, laquelle est en mesure d'interagir de façon au moins essentiellement spécifique avec le brin d'ADN unitaire du gène mutant complémentaire du brin sonde (brin complémentaire) ;
(d) un moyen de blocage spécifique du type sauvage, inhibant la liaison de la sonde capteur au gène de type sauvage ;
la teneur en première amorce (b) dans la composition étant plus grande que la teneur en deuxième amorce (c),
sachant que le moyen de blocage est choisi de telle sorte que le moyen de blocage présente, en rapport avec la zone du brin unitaire d'ADN du gène de type sauvage laquelle correspond au tronçon de gène du gène mutant présentant la mutation, une plus haute spécificité en comparaison du gène mutant correspondant.

15. Utilisation de la composition selon la revendication 14 pour la détection d'au moins une mutation dans un gène, de préférence dans un gène codant pour une protéine se trouvant en relation avec une affection tumorale et/ou cancéreuse, le gène présentant la mutation (gène mutant) étant présent ensemble avec d'autres gènes codant pour ladite protéine mais ne présentant pas de mutation (gènes de type sauvage), dans le cadre d'une réaction en chaîne asymétrique de polymérase (Polymerase Chain Reaction, c'est-à-dire PRC).
